# EUROPEAN PATENT APPLICATION

(11) **EP 1 457 561 A1**
(43) Date of publication of application: **15.09.2004**
(21) Application number: 02799421.9
(22) Date of filing: 26.09.2002
(51) Int. Cl.: C12N 15/53, C12N 9/02, C12P 23/00

(54) **BIOSYNTHETIC GENES OF BLAKESLEA TRISPORA BETA-CAROTENE THAT CODE FOR LYCOPENE CYCLASE/PHYTOENE SYNTHASE (CARRP) AND PHYTOENE DEHYDROGENASE (CARB)**

(30) Priority: 26.09.2001 ES 200102161
(71) Applicant: Vitatene, S.A., 24080 Leon (ES)
(72) Inventor: RODRIGUEZ SAIZ, Marta, E-24080 León (ES); MARCOS RODRIGUEZ, Ana, Teresa, E-24080 León (ES); DIEZ GARCIA, Bruno, E-24080 León (ES); DE LA FUENTE MORENO, Juan, Luis, E-24080 León (ES); BARREDO FUENTE, José, Luis, E-24080 León (ES)
(74) Representative: Elzaburu, Alberto de
(86) International application number: PCT/ES2002/000452
(87) International publication number: WO 2003/027293

(57) **Abstract**

Biosynthetic genes of β-carotene from *Blakeslea trispora* that code for lycopene cyclase / phytoene synthase (*carRP*) and phytoene dehydrogenase (*carB*). The *carRP* gene, characterized by the DNA sequence SEQ ID NO:1, codes for the amino acid sequence SEQ ID NO:3, which has the lycopene cyclase / phytoene synthase enzymes. For its part, the *carB* gene, characterized by the DNA sequence SEQ ID NO:2, codes for the amino acid sequence SEQ ID NO:4, which possesses phytoene dehydrogenase enzyme activity. In addition, (i) procedures for constructing plasmids for the expression of additional copies of the aforesaid genes and (ii) methods for the expression of heterologous genes under the control of the promoters of said genes, are described.

## Description

### Field of the invention

The present invention relates to two new genes of *Blakeslea trispora* designated *carRP* and *carB*. The gene *carRP* codes for the bifunctional enzyme lycopene cyclase / phytoene synthase, whereas *carB* codes for the enzyme phytoene dehydrogenase. Both genes are involved in the biosynthetic pathway of β-carotene of *B. trispora* (see scheme).

### State of the art

The carotenoids are pigments of an isoprenoid nature that are synthesized by certain bacteria, fungi and photosynthetic organisms. Owing to their beneficial effects on health and their attractive colors, the carotenoids are of considerable commercial importance as coloring matter and food additives. β-Carotene is a carotenoid whose chemical synthesis has been known since 1956. It has a molecular weight of 536.9, and a molecule (C₄₀H₅₆) with eleven conjugated double bonds. Its color is reddish-violet in the crystalline state, yellowish-orange in oily solution, and orange in aqueous dispersion. Synthetic β-carotene possesses the *all-trans* isomeric configuration, whereas the β-carotene obtained from various natural sources has various forms: *mono-cis, di-cis* and *poly-cis*.

The production of carotenoids by microbial biosynthesis is a classic example of competition between chemical and biological processes. Biotechnological processes show, among other things, the advantage of making it possible to obtain, in a simple way, the carotenoids of more complex structure, such as the conformational isomers that only exist in natural form. The industrial biotechnological processes for the production of β-carotene, competing with chemical synthesis, are based on the use of the alga *Dunaliella salina* and the fungus *B. trispora*. The production process with *B. trispora* involves carrying out a mixed fermentation of the (+) and (-) strains for achieving maximum production of β-carotene. This leads to the biosynthesis of trisporic acids, which induce the production of β-carotene. β-Carotene is produced both by the (+) strain and by the (-) strain, being metabolized by both to retinal and then to 4-dihydrotrisporol. The (+) strain uses 4-dihydrotrisporol as substrate for forming dihydrotrisporic acid and its methyl ester (methyl-4-dihydrotrisporate). For its part, the (-) strain metabolizes 4-dihydrotrisporol to trisporol. Finally, the methyl-4-dihydrotrisporate is converted to trisporic acid by the (-) strain and the trisporol is converted to trisporic acid by the (+) strain. This description of the biosynthesis of the trisporic acids is a simplification, since the process generates many co-metabolites, some of which are common to both strains (+) and (-), but others are specific to one of them. The relative amounts of these co-metabolites vary depending on the strains.

Despite the great biotechnological importance of the fungus *B. trispora*, scientific knowledge about the biosynthesis of β-carotene is scant, since: (i) basic procedures have not yet been described for its genetic manipulation, i.e. cloning of biosynthetic / regulatory genes and transformation and (ii) there is a lack of information on the characterization of enzymes involved in the biosynthesis of β-carotene. Nevertheless, the biosynthetic pathway of β-carotene (see scheme) has been described in phylogenetically related fungi such as *Phycomyces blakesleeanus* and *Mucor circinelloides* (Arrach N. et al. (2001) Proceedings of the National Academy of Sciences USA 98: 1687-1692; Velayos A. et al. (2000) European Journal of Biochemistry 267: 5509-5519). At least three enzymes are necessary for this biosynthesis: (i) phytoene synthase, which joins together two molecules of geranylgeranyl pyrophosphate generating phytoene, (ii) phytoene dehydrogenase, which introduces four double bonds in the phytoene molecule to synthesize lycopene, and (iii) lycopene cyclase, which, using lycopene as substrate, takes on the task of forming the rings located at both ends of the β-carotene molecule.

Analysis of mutants of *B. trispora* has led to the conclusion that the biosynthetic pathway of β-carotene in this fungus is similar to that described for *P*. *blakesleeanus* (Metha B.J. and Cerdá-Olmedo E. (1995) Applied Microbiology and Biotechnology 42: 836-838). In the case of *P. blakesleeanus*, the yellow color of its mycelium can be altered by mutation, giving rise to strains with mycelium colored red, white or various shades of yellow. The red mutants accumulate lycopene, whereas the white ones lack production of carotenoids or accumulate phytoene. Using complementation analysis, two genes have been identified called *carB* and *carRA*, whose products are probably organized in an enzyme complex in which the four dehydrogenations are catalyzed by four identical units of phytoene dehydrogenase and the two cyclizations are catalyzed by two identical units of lycopene cyclase (Candau R. et al. (1991) Proceedings of the National Academy of Sciences USA 88: 4936-4940). Both the *carB* gene (Ruiz-Hidalgo M.J. et al. (1997) Molecular and General Genetics 253: 734-744) and the *carRA* gene (Arrach N. et al. (2001) Proceedings of the National Academy of Sciences USA 98: 1687-1692) have been cloned and characterized. The *carRA* gene has two different domains: (i) the domain R, which is located nearer to the 5' end and codes for the lycopene cyclase activity and (ii) the domain A, which is responsible for the phytoene synthase activity. Moreover, the *carB* gene of *P. blakesleeanus* has been expressed in *M*. *circinelloides* (Ruiz-Hidalgo M.J. et al. (1999) Current Microbiology 39: 259-264).

The genes *carB* (Velayos A. et al. (2000) Planta 210: 938-946) and *carRP* (Velayos A. et al. (2000) European Journal of Biochemistry 267: 5509-5519) of *M. circinelloides* have also been cloned and characterized. The *carRP* gene codes for a bifunctional enzyme lycopene cyclase / phytoene synthase with two domains: (i) the domain R, which is situated nearer the 5' end and codes for the lycopene cyclase activity and (ii) the domain P, located at the 3' end and responsible for the phytoene synthase activity. The domain R is functional even in the absence of the domain P, whereas the domain P requires the presence of the domain R for proper functioning.

### Detailed description of the invention

The present invention describes for the first time the *carB* and *carRP* genes of *B*. *trispora*. As has been described previously for *P. blakesleeanus* and *M*. *circinelloides,* the *carB* gene codes for phytoene dehydrogenase and the *carRP* gene codes for a bifunctional enzyme lycopene cyclase / phytoene synthase with a domain R (nearer the 5' end and coding for the lycopene cyclase activity) and a domain P (located at the 3' end and responsible for the phytoene synthase activity). Concretely, the product encoded by *carB* carries out the conversion of phytoene to lycopene and the bifunctional enzyme encoded by *carRP* catalyzes both the biosynthesis of phytoene from geranylgeranyl-PP and the conversion of lycopene to β-carotene. Both genes: (i) are involved in the biosynthetic pathway of β-carotene, (ii) are contiguous in the genome and (iii) are expressed under the control of a bidirectional promoter located between the two. This bidirectional promoter permits gene expression in one direction or in the opposite direction. Mutants lacking either of these two genes are not capable of producing β-carotene, but accumulate the corresponding biosynthetic intermediates (see scheme).

*B*. *trispora* is a fungus that is of considerable industrial importance for the biotechnological production of β-carotene. In fact, this process is becoming competitive with the synthetic process used industrially at present. The aforementioned genes *carRP* and *carB* can be used, for example, for (i) improving the yield of β-carotene by increasing its expression and (ii) modifying the biosynthetic pathway of β-carotene by generating strains capable of biosynthesis of other carotenoids. Gene expression of *carRP* and *carB* can be increased by inserting extra copies of said genes in *B. trispora*, either in their natural state or expressed under the control of strong promoters. Modification of the biosynthetic pathway of β-carotene can be achieved, for example, by inactivating the lycopene cyclase activity by manipulation of the *carRP* gene. In this way, strains would be generated that are able to produce lycopene, a carotenoid of considerable industrial and commercial interest.

The scheme on the next page shows the genes and enzymes involved in the biosynthetic pathway of β-carotene of *B*. *trispora*. The phytoene synthase activity joins together two molecules of geranylgeranyl pyrophosphate, generating phytoene. The phytoene dehydrogenase activity introduces four double bonds in the phytoene molecule to synthesize lycopene. The lycopene cyclase activity, using lycopene as substrate, forms the rings located at both ends of the β-carotene molecule.

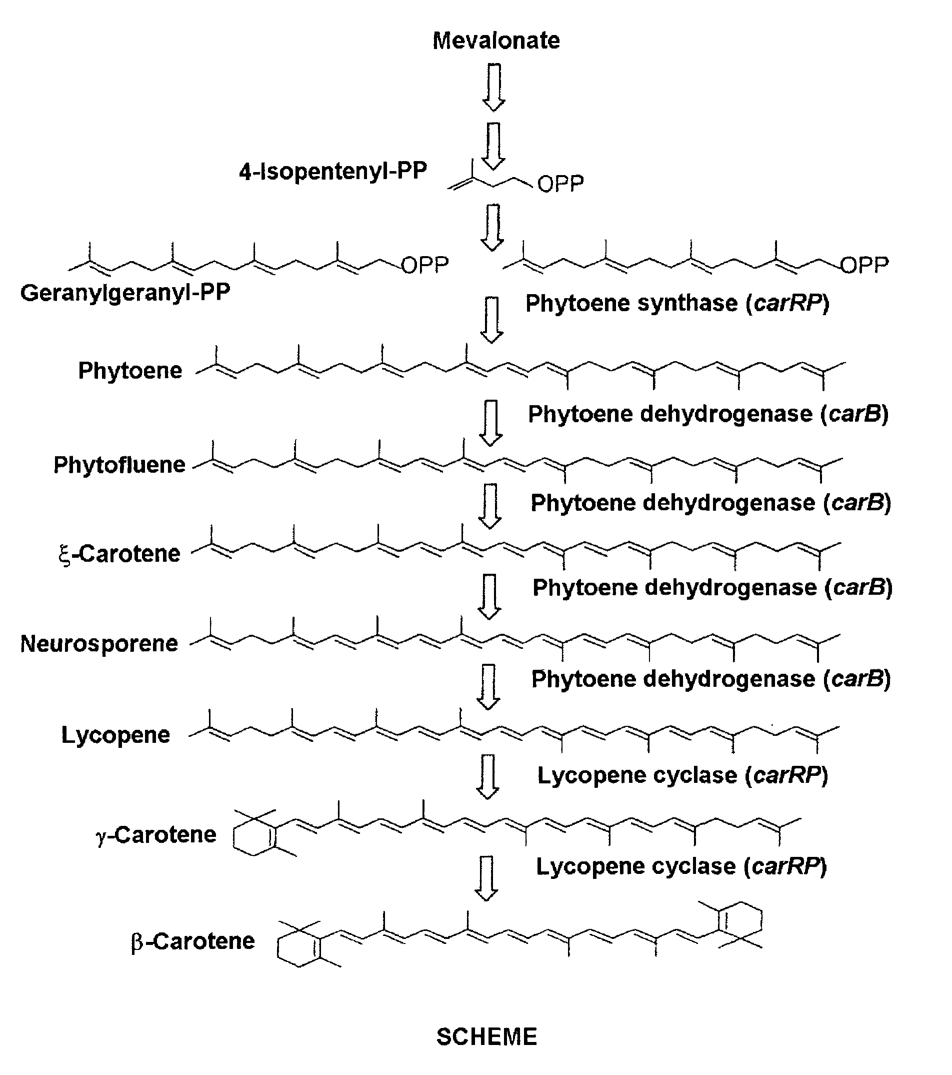

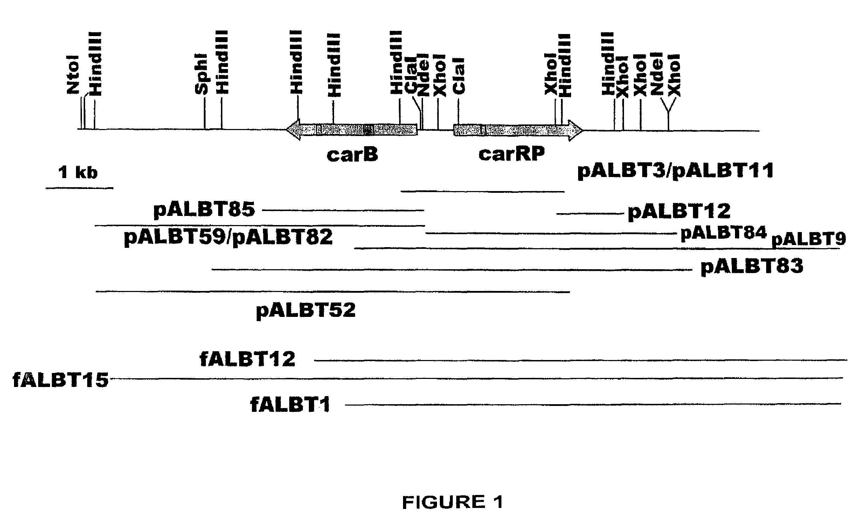

The genomic DNA of the fungus *B*. *trispora* was used for constructing a gene library in the phage vector λ-GEM12. For this, partial digestion was effected with the restriction endonuclease *Sau*3AI and the resulting fragments were joined to λ-GEM12, as described in Sambrook J. et al. (1989) Molecular Cloning: A Laboratory Manual, Cold Spring Harbor Laboratory, Cold Spring Harbor, New York, USA. The genes *carRP* (lycopene cyclase / phytoene synthase) and *carB* (phytoene dehydrogenase) were cloned by screening the said gene library of *B*. *trispora* using a probe corresponding to the *carRP* gene of *Mucor circinelloides.* The probe was obtained by PCR (polymerase chain reaction) using primers designed on the basis of the sequence of the *carRP* gene of *Mucor circinelloides* (Velayos A. et al. (2000) European Journal of Biochemistry 267: 5509-5519). In this way a 560-bp DNA fragment was amplified and was used for scanning the gene library, isolating 4 recombinant phages designated fALBT1, fALBT4, fALBT12 and fALBT15 (Fig. 1). Said clones were analyzed with a series of restriction endonucleases and then a 2.4-kb *Hind*III fragment was subcloned in plasmid vectors of *E*. *coli*. The restriction map of the aforesaid fragment is shown in the Scheme. Next, the sequence of the 2430 bp included in the aforementioned *Hind*III fragment was determined, and two incomplete open reading frames (ORFs) were found, which were transcribed in opposite directions. On the basis of their similarity with sequences present in databases they were designated *carRP* (SEQ ID NO:1) and *carB* (SEQ ID NO:2). Subsequent subcloning and sequencing of the DNA regions adjacent to the ends of the aforesaid 2.4 kb fragment *Hind*III made it possible to complete the nucleotide sequence of both ORFs.

The ORF corresponding to the *carRP* gene has a length of 1894 bp and is interrupted by a 70-bp intron located between positions 406 and 475. Said ORF codes for a protein of 608 amino acids, 69,581 Da and an isoelectric point of 7.78. Comparison of its deduced amino acid sequence (SEQ ID NO:3) with the SwissProt database shows great similarity with genes that code for the lycopene cyclase / phytoene synthase enzymes of *M*. *circinelloides* (67%), *P. blakesleeanus* (55%) and *Neurospora crassa* (29%) (Velayos A. et al. (2000). European Journal of Biochemistry 267: 5509-5519; Arrach N. et al. (2000). Proceedings of the National Academy of Sciences USA 98: 1687-1692; Schmidhauser T.J. et al. (1994). The Journal of Biological Chemistry 269: 12060-12066).

The ORF corresponding to the *carB* gene has a length of 1955 bp and is interrupted by two introns of 141 bp and 68 bp, located between positions 594-734 and 1584-1651 respectively. This ORF codes for a protein of 582 amino acids, 66,426 Da and an isoelectric point of 6.9. Comparison of its deduced sequence of amino acids (SEQ ID NO:4) with the SwissProt database shows great similarity with genes that code for the phytoene dehydrogenase enzyme in *M. circinelloides* (80%), *P*. *blakesleeanus* (72%) and *Neurospora crassa* (48%) (Velayos A. et al. (2000). Planta, 210: 938-946; Ruiz-Hidalgo et al. (1997). Molecular and General Genetics 253: 734-744; Ruiz-Hidalgo et al. (1999). Current Microbiology 39: 259-264; Schmidhauser et al. (1990). Molecular and Cellular Biology 10: 5064-5070).

A 6.9-kb DNA fragment that includes both genes (*carRP* and *carB*) was subcloned, using suitable restriction targets, in plasmid vectors that can be incorporated in one or more copies in the genome of *B*. *trispora*, which permit expression of these genes in the host fungus. Similarly, the promoters of the *carRP* and *carB* genes can be obtained (using suitable restriction targets or by PCR) and used for expressing homologous and heterologous genes in *B*. *trispora*. The following examples describe the expression of the phleomycin resistance gene (*ble*^{*R*}) of *Streptoalloteichus hindustanus* (Drocourt D. et al. (1990). Nucleic Acids Research 18: 4009) under the control of the promoters of the *carRP* and *carB* genes. As was described earlier, the promoters of the *carRP* and *carB* genes can be used for the correct expression of heterologous genes in *B*. *trispora* or for the overexpression of homologous genes that are weakly transcribed.

The expression in *B*. *trispora* of biosynthetic genes of xanthophylls can give rise to transformants capable of biosynthesizing carotenoids such as zeaxanthin, canthaxanthin, astaxanthin or new carotenoids. In addition, by means of gene interruption, it would be possible for the biosynthetic pathway of β-carotene to be blocked, obtaining transformants capable of producing, for example, lycopene as the end product.

### Deposit of microorganisms under the Budapest Treaty.

Two strains of *Escherichia coli* bearing the plasmids pALBT9 (which contains the *carRP* gene) and pALBT52 (which contains the *carB* gene) were deposited, in accordance with the provisions of the Budapest Treaty, in the Spanish Type Culture Collection (CECT), Department of Microbiology of the Biology Faculty of the University of Valencia, Campus de Burjasot, 46100 Burjasot, Valencia, (Spain), with the numbers CECT 5982 and CECT 5981 respectively on 09.17.01.

The following examples describe the present invention in detail and without limitation.

### EXAMPLE 1

### Construction of gene libraries of the (+) and (-) strains of B. trispora.

Gene libraries of the (+) and (-) strains of *B. trispora* were constructed in the following way: A total of 300 µg of whole DNA was digested partially with 20 units of *Sau*3AI in a reaction volume of 600 µl at 37°C and 3 aliquots of 200 µl were collected at 45 seconds, 1 minute and 2 minutes respectively, digestion being stopped with cold EDTA 20 mM. After confirming the digests in 0.7% agarose gel, they were mixed, heated at 68°C for 10 minutes, left to cool slowly to room temperature and placed on a sucrose gradient (10-40%) of 38 ml. This gradient was centrifuged at 26,000 rpm for 24 hours at 25°C, after which 0.5-ml aliquots were collected, and 10 µl of each one was analyzed in 0.4% agarose gel. The aliquots whose DNA had a size between 18 and 22 kb were mixed and then diluted with distilled water to approximately 10% sucrose. Then the DNA was precipitated with ethanol, resuspended in 50 µl of TE buffer and 3 µl of this last-mentioned solution was analyzed in 0.4% agarose gel. It was confirmed in this gel that the size of the DNA fragments was correct and that their concentration was approximately 50 ng/µl.

In parallel, the DNA of the bacteriophage λ-GEM12 (Promega) was prepared by methods described previously (Sambrook J. et al. (1989) Molecular Cloning: A Laboratory Manual, Cold Spring Harbor Laboratory, Cold Spring Harbor, New York, USA). 50 µg of DNA from the bacteriophage was digested with the endonucleases *Bam*HI and *Eco*RI at 37°C for 2 hours. The double digest was extracted with phenol/CIA and CIA, precipitated with ethanol and resuspended in 50 µl of TE buffer. After collecting a 2-µl aliquot, MgCl₂ was added to the rest, to 10 mM, then it was incubated at 42°C for 1 hour to promote circularization of the arms of the vector on its cohesive ends. A 2-µl fraction was collected again and was analyzed together with the previous one in 0.5% agarose gel to confirm correct circularization on the cohesive ends and determine its approximate concentration (around 100 ng/µl).

Next, a series of ligations was carried out using 0.25 µg of insert and quantities of vector between 0.25 and 0.75 µg, varying the insert/vector ratio. The reactions were incubated at 12-14°C for 16 hours. Encapsidation of the recombinant phage DNA produced after ligation was performed with 'Packagene' (Promega) *in vitro* packaging extracts. With the resultant from the encapsidation reaction resuspended in 500 µl of SM, infections of *E*. *coli* NM538 (Promega) were carried out to determine the number of phages present and of *E. coli* NM539 (Promega) with the aim of determining the percentage of recombinant phages. *E. coli* NM539 is a lysogenic strain of the P2 phage and only produces lysis plaques when the phage infecting it lacks the dispensable central region.

The titer of the gene libraries constructed proved to be as follows: 125,000 pfu for *B*. *trispora* (+) and 50,000 pfu for *B. trispora* (-). In both cases around 80% of the phages were carriers of an exogenous DNA fragment.

### Example 2

### Cloning and characterization of the carB and carRP genes of B. trispora.

The two gene libraries were transferred to nitrocellulose filters and were scanned with a 560-bp probe corresponding to the *carRP* gene of *M*. *circinelloides.* This probe was obtained by PCR amplification using the primers #61 (SEQ ID NO:5) and #62 (SEQ ID NO:6), which were designed as a function of the DNA sequence of the *carRP* gene of *M*. *circinelloides* (Velayos A. et al. (2000) European Journal of Biochemistry 267: 5509-5519). The process for selecting positive phages was carried out in accordance with hybridization methods previously described (Sambrook J. et al. (1989) Molecular Cloning: A Laboratory Manual, Cold Spring Harbor Laboratory, Cold Spring Harbor, New York, USA). Once the process of prehybridization, hybridization, washing and autoradiography was completed, 4 clones were selected, designated fALBT1, fALBT4, fALBT12 and fALBT15, which produced positive signals with probe DNA. The DNA that includes the *carRP* and *carB* genes claimed in this invention can be obtained by digesting the phages fALBT4 or fALBT15 (Fig. 1) with suitable restriction enzymes using standard techniques (Sambrook J. et al. (1989) Molecular Cloning: A Laboratory Manual, Cold Spring Harbor Laboratory, Cold Spring Harbor, New York, USA). DNA fragments containing the sequences SEQ ID NO:1 and SEQ ID NO:2 can be obtained with one or more restriction enzymes (Fig. 1) and subcloned in a suitable vector (for example pBluescript).

A 2.4-kb *Hind*III DNA fragment obtained from the fALBT1 phage (Fig. 1) was subcloned in the *Hind*III restriction site of the plasmid pBluescript I KS (+) in the two possible orientations. The resulting plasmids were designated pALBT3 and pALBT11. These plasmids were subjected to sequence deletions with the 'Erase-a-base' kit (Promega) in accordance with the manufacturer's instructions. The sequence reactions of the deleted clones were carried out with the 'Dye Terminator Cycle Sequencing Ready Reactions Kit' and the DNA fragments were separated in an ABI PRISM automatic sequencer (Perkin Elmer) following the manufacturer's instructions. In this way we obtained the sequence of nucleotides, on both chains, of a total of 2430 bp. Using the Geneplot program (DNASTAR), two incomplete ORFs were identified which corresponded to the *carRP* and *carB* genes. With the aim of completing the nucleotide sequence of the two genes, we carried out subcloning and subsequent sequencing of the DNA regions adjacent to the aforementioned *Hind*III fragment. Starting from the fALBT1 phage, a 1.0-kb *Xho*I fragment was subcloned in pBluescript I KS (+) (Fig. 1), which includes the 3' end of the *carRP* gene, giving rise to the pALBT12 plasmid. Sequencing for both chains of the fragment included in said plasmid made it possible to complete the nucleotide sequence of the *carRP* gene.

In addition, with the aim of determining the sequence of the 3' region of the *carB* gene, the subcloning of a 5.1-kb *Cla*I-*Not*I fragment obtained from fALBT15 was performed in pBluescript I KS (+). The plasmids obtained (both orientations), designated pALBT59 and pALBT82, were submitted to sequence deletions with the 'Erase-a-base' kit (Promega) according to the manufacturer's instructions. The sequence reactions of the deleted clones were carried out with the 'Dye Terminator Cycle Sequencing Ready Reactions Kit' and the DNA fragments were separated in an ABI PRISM automatic sequencer (Perkin Elmer) following the manufacturer's instructions.

The complete nucleotide sequence of the *carRP* gene revealed an ORF of 1894 bp, interrupted by an intron of 70 bp, situated between positions 406-475 and flanked by the following intron/exon splicing sequences: 5' (g/GTATGCA) and 3' (TTAG/c). It is a question of conserved sequences when they are compared with the consensus sequences described for fungi: 5' (g/GTAHGTYW) and 3' (MYAG/g). This gene codes for a polypeptide of 608 amino acids with a deduced molecular weight of 69,581 Da and an isoelectric point of 7.78. The protein deduced from the sequence (SEQ ID NO:3) shows great similarity with the enzymes with lycopene cyclase / phytoene synthase activity of *M*. *circinelloides* (67%), *P. blakesleeanus* (55%) and *N*. *crassa* (29%) described in the databases (Velayos A. et al. (2000). European Journal of Biochemistry 267: 5509-5519; Arrach N. et al. (2000). Proceedings of the National Academy of Sciences USA 98: 1687-1692; Schmidhauser T.J. et al. (1994). The Journal of Biological Chemistry 269: 12060-12066).

The *carB* gene, 1955 bp in length, is interrupted by two introns of 141 and 68 bp, located between positions 594-734 and 1584-1651 respectively, and flanked by conserved fungal intron/exon splicing sequences [5' (g/GTAAGTA) and 3' (ATAG/t) for the 141-bp intron and 5' (g/GTAATAC) and 3' (GTAG/t) for the 68-bp intron] similar to those described for the *carRP* gene. The *carB* gene codes for a polypeptide of 582 amino acids with a deduced molecular weight of 66,426 Da and an isoelectric point of 6.9. The protein deduced from the sequence (SEQ ID NO:4) shows great similarity with the enzymes with phytoene dehydrogenase activity of *M*. *circinelloides* (80%), *P. blakesleeanus* (72%) and *N*. *crassa* (48%) described in the databases (Velayos A. et al. (2000). Planta, 210: 938-946; Ruiz-Hidalgo et al. (1997). Molecular and General Genetics 253: 734-744; Ruiz-Hidalgo et al. (1999). Current Microbiology 39: 259-264; Schmidhauser et al. (1990). Molecular and Cellular Biology 10: 5064-5070).

### Example 3

### Construction of plasmids for heterologous expression in B. trispora.

The pALFleo7 plasmid (Díez B. et al. (1999) Applied Microbiology and Biotechnology 52: 196-207) was used as the starting point for constructing heterologous expression plasmids in *B. trispora*. Said plasmid contains the phleomycin resistance gene (*ble*^{*R*}) of *S. hindustanus* (Drocourt D. et al. (1990). Nucleic Acids Research 18: 4009) with a restriction site for *Nco*I located on the ATG translation start codon and a restriction site for *Bam*HI preceding the *Nco*I. pALFleo7 also possesses the *trpC* gene terminator of *Aspergillus nidulans* (T*trpC*). The cluster formed by the *ble*^{R} gene and T*trpC* was purified by double digestion of pALfleo7 with *Bam*HI plus *Bgl*II and was subcloned at the *Bam*HI site of pBluescript I KS(+). The resulting plasmid was designated pALfleo8. Next, two *Nco*I sites were inserted in the ATG translation start codons of the *carB* and *carRP* genes, by directed mutagenesis using the 'QuikChange™ Site-Directed Mutagenesis Kit' (Stratagene). For inserting the *Nco*I cutting site in the *carB* gene, the following oligonucleotides were designed, represented by SEQ ID NO:7 and SEQ ID NO:8. For inserting the *Nco*I cutting site in the *carRP* gene, the oligonucleotides represented by SEQ ID NO:9 and SEQ ID NO:10 were designed. In both cases pALBT3 was used as template for PCR amplification. Once the corresponding mutations were inserted, the pALBT56 plasmid was obtained.

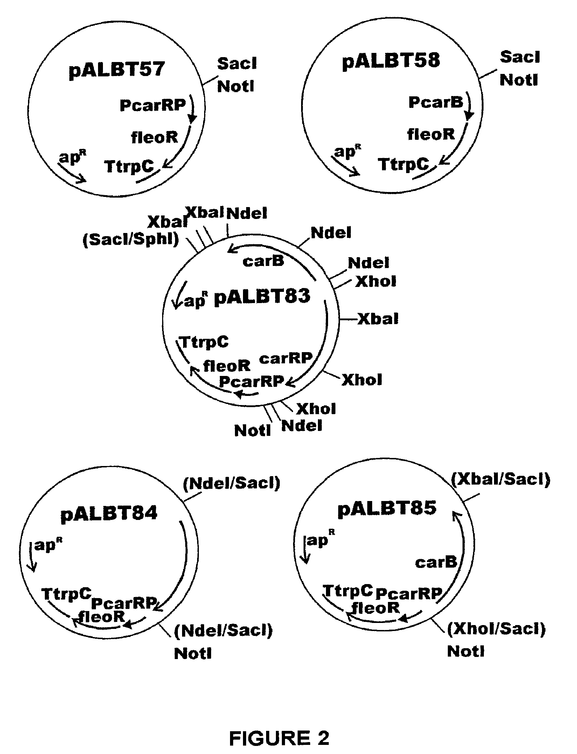

Starting from the pALBT56 plasmid, a 0.6-kb *Nco*I fragment was purified, with the 'Quiaex II' kit (Quiagen), corresponding to the bidirectional promoter of the *carB* and *carRP* genes (P*carB*-P*carRP*). This fragment was joined to the pALfleo8 plasmid, previously digested with the endonuclease *Nco*I, obtaining two different plasmids: pALBT57 (which expresses the *ble*^{*R*} gene under the P*carRP* promoter) and pALBT58 (which expresses the *ble*^{*R*} gene under the P*carB* promoter). The plasmids pALBT57 and pALBT58 (Fig. 2) permit the heterologous expression of the *ble*^{R} gene of *S. hindustanus* in *B. trispora*.

### Example 4

### Construction of plasmids for inserting additional copies of the carRP and carB genes in B. trispora.

With the aim of inserting additional copies of the *carRP* and *carB* genes in *B*. *trispora*, the plasmids pALBT83, pALBT84 and pALBT85 were constructed (Fig. 2). In all three cases, the pALBT57 plasmid described previously was used as the starting point. The construction of pALBT83 was carried out in the following way: fALBT4 was digested with the endonuclease *Sph*I, then its ends were blunted with the Klenow fragment of DNA polymerase I from *E. coli* (without addition of dNTPs for promoting the action of its exonuclease activity) and finally was digested with the endonuclease *Not*I. Next, a 6.9-kb fragment was purified, with the 'Quiaex II' kit (Quiagen), which contains the *carB* and *carRP* genes. Furthermore, the pALBT57 plasmid was digested with the endonuclease *Sac*I, then incubated with the Klenow fragment of DNA polymerase I to obtain blunt ends (without addition of dNTPs for promoting the action of its exonuclease activity) and finally was digested with *Not*I. The compatible ends of the plasmid and of the insert were joined and the result of the ligation was transformed by electroporation into *E. coli* DH5α. The transformants were selected in LB medium (Sambrook J. et al. (1989) Molecular Cloning: A Laboratory Manual, Cold Spring Harbor Laboratory, Cold Spring Harbor, New York, USA) for ampicillin resistance (present in the pALBT57 vector). The desired construction was selected by means of restriction analysis, obtaining the pALBT83 plasmid (Fig. 2).

Construction of pALBT84 was carried out in the following way: pALBT9 was digested with the endonuclease *Nde*I and then its ends were blunted with the Klenow fragment of DNA polymerase I of *E*. *coli*. Next, a 3.5 kb fragment containing the *carRP* gene was purified, with the 'Quiaex II' kit (Quiagen). In addition, the pALBT57 plasmid was digested with the endonuclease *Sac*I and was then incubated with the Klenow fragment of DNA polymerase I to blunt the ends (without addition of dNTPs for promoting the action of its exonuclease activity). The blunt ends of the plasmid and of the insert were joined and the result from ligation was transformed by electroporation in *E. coli* DH5α. The transformants were selected in LB medium (Sambrook J. et al. (1989) Molecular Cloning: A Laboratory Manual, Cold Spring Harbor Laboratory, Cold Spring Harbor, New York, USA) for ampicillin resistance (present in the pALBT57 vector). The desired construction was selected by restriction analysis, obtaining the pALBT84 plasmid (Fig. 2).

Construction of pALBT85 was carried out in the following way: pALBT52 was digested with the endonucleases *Xho*I-*Xba*I and then its ends were blunted with the Klenow fragment of DNA polymerase I of *E. coli*. Next, a 2.6-kb fragment containing the *carB* gene was purified, with the 'Quiaex II' kit (Quiagen). In addition, the pALBT57 plasmid was digested with the endonuclease *Sac*I and was then incubated with the Klenow fragment of DNA polymerase I to blunt the ends (without addition of dNTPs for promoting the action of its exonuclease activity). The blunt ends of the plasmid and of the insert were joined and the result from ligation was transformed by electroporation in *E. coli* DH5α. The transformants were selected in LB medium (Sambrook J. et al. (1989) Molecular Cloning: A Laboratory Manual, Cold Spring Harbor Laboratory, Cold Spring Harbor, New York, USA) for ampicillin resistance (present in the pALBT57 vector). The desired construction was selected by restriction analysis, obtaining the pALBT85 plasmid (Fig. 2).

With the aim of clearly defining the invention, a total of two diagrams are presented. The abbreviations *Bam*HI, *Hind*III, etc. are conventional abbreviations for restriction endonucleases. The approximate length of the DNA fragments is shown in kilobases (kb) according to the sizes determined by agarose gel electrophoresis. The diagrams do not show all of the restriction sites present. The DNA of the invention can be inserted in any suitable vector by various methods: direct ligation of cohesive ends, use of homopolymeric adhesives, by means of adapting or ligating molecules, etc.

### Detailed description of the diagrams

**Fig. 1.** Restriction map of the region of the genome of *B. trispora* that contains the *carB* and *carRP* genes. The arrow indicates the direction of transcription of each of them, and the rectangles indicate the presence of introns. The lines in the lower part of the diagram represent the DNA fragments of *B*. *trispora* cloned in the phage vector λ-GEM12 (fALBT) or in plasmids (pALBT). The nucleotide sequence of this DNA fragment is described in SEQ ID NO:1 and SEQ ID NO:2.
**Fig. 2.** Physical map of the plasmids pALBT57, pALBT58, pALBT83, pALBT84 and pALBT85. pALBT57 consists of pBluescript I KS (+) with a 1.7-kb *Hind*III-*Bam*HI insert that includes the phleomycin-resistance construction P*carRP-ble*^{*R*}-T*trpC*. pALBT58 consists of pBluescript I KS (+) with a 1.7-kb *Hind*III*-Bam*HI insert that includes the phleomycin-resistance construction P*carB-ble*^{*R*}-T*trpC*. pALBT83 is formed by pALBT57 with a 6.9-kb *Sph*I-*Not*I insert that includes the *carB* and *carRP* genes. pALBT84 consists of pALBT57 with a 3.5-kb *Nde*I insert that includes the *carRP* gene. pALBT85 is pALBT57 with a 2.6-kb *Xho*I-*Xba*I insert that includes the *carB* gene.

SEQUENCE LIST

<110> Antibióticos, S.A.U.

<120> "Biosynthetic genes of β-carotene of Blakeslea trispora
that code for lycopene cyclase / phytoene synthase
(carRP) and phytoene dehydrogenase (carB)"

<130> P-99669

<160> 10

<210> SEQ ID NO:1

<211> 8786

<212> DNA

<213> Blakeslea trispora

<220> CDS

<221> carRP

<222> 5688...7581

<220> Exon

<222> 5688...6092

<220> Exon

<222> 6163...7581

<223> Immediate source: plasmids pALBT9 (CECT 5982) and
pALBT52 (CECT 5981).

<223> β-Carotene biosynthesis genes.

<400>

GGGCTCACTT GTATCATCAC TGTTGTTAAT AAATTGTTGA GAATAAAGCT TGGATTGTAT 60
ACGACTGAGC CAGTTGGAGC CACTTGTAGG CCTTGTGGGT GATACAACAG CAGGCTTTGA 120
TACAGTGGTA TTTATTTCTG GCCTAAACTT CAATATACTT TTTGGACTGT TCATGCCTTG 180
ATATAACAAA ATAAAATAAA AAGGGACCTT GTACACTGAA AGCCAAATGA ATAAAATAAA 240
AAATAAAAAA TAAAAAGTTA TTTGCAGAAA CCCAAAAAGC CCGAAATTTC TTCTAAAAGA 300
AAGAAAATAA AAAAAAAGAG AGACTTACTT TTAGGCGATC AACAGTCTTT TTTTTTTGCT 360
TGAGCTTCTT TTAAGCTTTA CATTTAGGCA CTTTTATAAT TGCTGTATTG AATATGACAT 420
CTATCTTTTT TAACTTGAAC GATTTGTAGC TGTTTTCTCT TGACAATGTT AATTTTAAAG 480
CATGATTTTT TTTCTGAAAA AATAAAGACC GCTTACATAA TACAAAAGTC AGAATTGTAT 540
GGCTATTTTA TCCTGTCTCT TCTTTTCAGG AACTCAACCA TCTCCTTGTC TTTATTGTCT 600
GAGAATGCTT TATCTGACTC TTATTAATCC TTTGAGAAAC CAAAAATGGG GTGGATTGAG 660
ACTTGGACTC GCTTTGTGCA TTTGCTCCTT CTGTTCGATC ATTATTGAGC AATGTCATAC 720
AAACATTCAA TGACAAGCAA CATCAAACTA GGAGACAGAT TAAGCAAGCA GATGATTTAT 780
AAAGGGCTAC TCTGCTCATT GATTTACATG CTTTAGCATC ATGAATTGTG TTGCTTCCAT 840
TCCAAAATAG ATGTCTTTTT CTCTTTCCAC TTGCCTCTTT CTTTTTTTGG CTATTCAGCA 900

TAATGCAAGA GTAGCTATTC CTCAACCCAT CAGTAATAAG TGAATGGCAC GGTAGCATTG 960
TGTTTGTATG TTTTCTATCT GCGTTTTGAT TTTCGAATCT CATCTTTAGG ATAGTATCTT 1020
GAGATTGTAT TGATTTTGCC TGATACACTT TTGTTTTTGT GAATATGAGC TTCAGGGAAG 1080
TGATCTGTTT AAGTACAATT GAAATAAAGT GATAGTCTTT GAGGTTTGGT ATATGGCTTT 1140
CTAAAGCATG GACAGAGCCA TGACAAAAAA AAGGGCGGTA TAAAGCCTCA GATAACTCCC 1200
TCTCTTGCAC TCTGACAAAT GTAGTACAGG CTTATTGCCA ATGGAATGAC TCAATAGATG 1260
GCTAAATGTG AAGAAAAACG TATTGGAAAA GCCTACAAAA CTTTCCTTAA TAAACATCAT 1320
GGCCATCTTT TTTGAAAGAT TTCAAAGACT AGGTTAAAAA TACCATTACT ATCATCTTGC 1380
TTCTAGTCCT CTGACTATTT CTTTGTTTTC AACAACACGT GTAGGAAGGA CAAGAAAGAT 1440
GTAAGCGACA GAATAGCTTA GTTTTACTAT ACTCTACTTC TTTTTTTTTT GTAGTTTTCC 1500
ATCTTCCTTG CTTTGAAGAT AGACAATTTT ACTCACACAA TTTCTTTTTC GTTAAAGCTG 1560
ATAGCTTGAA TATCATCATA TCAAACAGAA GACATATATC GCTAAATAGT ACTTGCTATA 1620
ACAGAGAAAG CGATCCATGT GCAACTCTGA TGCTTATCGC CGTAGACGAC TTTCCTTAAA 1680
AAAAACTGAC AAACACCGTA GAATGTGACA CATACACACA ACAATACTGT TGCTCAAAAC 1740
TTTAACTAAT AGTTAAAGTA CAATGAAAAT ATATTGCAGT CTAGAATGAT AACAATTTTG 1800
CTTTAACGAT GTGGTGCAGT CTTAATTCAA AGCTCAAAGA AAAGAAAAAG AATTTGCTAG 1860
CTATCATGAG CTAAATCTCT GTTTTTCTTG AAACAATCTT ACAAAGAAAA GCTATATTGC 1920
TTGACAAAGG GCAAATCCAC CGAGATTCTT TTACTGCATG CCCGAATAAA AAGGGAGAGG 1980
AAGAGGAGTA TGTTACACTT GAATGTATTT TTGAGGAAGC ATCCGCATTA ATTTGGTGTT 2040
ATAAACACTG AGTAATCATG TTATTTGAAG ACTGAACCTT TACCAAAAAG GTCTTGTAGA 2100
TCGCTTGTTG CAATTGAGAT GGGTTAAAAA TAAGTCTAAA GTTAAGATAA GCACAATGAA 2160
AAGAACGTTT TTATTTTCTA TCAGGCAAAG TAAAACCACT TTTCTAGATG TGGCAATAAG 2220
CAATCAAGCC AAAGGGAGAA AAAAGCTTAT CAAAGCTATG GCTTTCAAGA GAATAGTAAT 2280
TTAGGTACTA CACAAAGCCA CTGTTTATGC TTCTTTGCAA TATCAACAAA GAGACATTGT 2340
GTCTGTTGAA ATGTTTTGTT TGACATGTTT AATCAGATCA AGTGAGGATG CTTTACTCTT 2400
TGGTTTAGTA AAAGAAACAC ACCAGCAACT CCGGTGAATG TTATGATTAT GACGTTTCAA 2460
ACGAAAAATC TCTATTTTCG TTTTAAGGTT AGTCCTTTTA GAATACCGTT TTTTTTTTTA 2520
CCATTTCATT GTCTTGAAAA CCCTCCCAAG CTAATGATTA TTTTCTTTTT TTACGCAAGA 2580
CTCGTATCAC TCACCTACCT TACAGACGTG TTTTGCTTTT TTGGATAATG CTGTGCTTGA 2640
TCTATGTATG GCTCCTTTGC CTTATTTTTA AAAAGAAATG TTTGCTAGGA TTGATTTTTA 2700
ATGGTTACTC TCAATCAAAT ACCACATTTA GTAGAAACAA AATTTGTGCA TATCATAATA 2760
AAACTAAATT CGATTATTTT TTCTAAAATC AGGATAATTT GTTTTTCCAA TATTTGTTTG 2820
TAGAATTGTC TGTCCTACCA AACAATTCAG TTTTCTATTT GCGTCGAGTC ATTTATTTTG 2880
GGTTTCTTTG TTTGAGCTGA TTCTGATACA CATGTGAATT GTCTTTTTAG ACACTATTCT 2940
AGAATTCATT CCATTCGAAA GGATCAACAT ACACCAATTT AATGACGTGC TAGATAATGG 3000
ATACAAATAT ACGCACAAAA AAAGAAAGAA TTCTATGATC AAAGAGAACG CAGACACAGA 3060
GTGATACATT TAAATGGTTA AGTTCATATG ATGTTAAAAT GGTAGCTTTA TTATTGAACT 3120
AAATGCGAAT ATCGTTGCTG TTTTGTCCTT GGAAAACGTT AGGTAAAAGT TGGTTAATGA 3180
AAGAAGCAGG AGTTGTAGTA TCATCTCTTG GGAAGAAATA GAAAAAGAGG AAAGTAACAA 3240
AGTAACAAGC AAGACAATAA TAGATCCAAT GGCTTTCGGT CTTACGAGTT TGTTCAGGAG 3300
CATACTTCTT TTGGCTATCT TGTAACTTTC TTGGTAAGGG ATTCTGGCCA AAGCTTTTAC 3360
AGACTTGGTC GGAAGTAAGC TTACTTCCAG CAAGAACGAT AGGAACACCA GTACCTGGAT 3420
GTGTACTACA AAGAAAAGAG AAATGAGTAC GTGCGTTATT AAAAAAAAGA AAAAAAGAGG 3480
GCAAAAGTAT TACCTAGCTC CGACAAAGAA AAGATTATCA TAACGGTTTG TGGAATCCTT 3540
GGTACTAGGT CTGAACCAGA GAACTTGGAA CACATCATGA GAAAGACCAA GAATAGAACC 3600
TCTCCAAAGG TTAAACTTGC TTTGCCAAAC ACTAGGATCA TTCACTTCTT CATGTTCAAT 3660
CAAATTAGCA AAGTTGTTTA CTCCCAAACG ACGTTCGATA ACTTCCAGAA CCATCTTGCG 3720
TGCACGGTTT ACCAACTCAG GATAATTTTC TTCAGCACTG TTTCCTGTCT TACTCTTCAT 3780
ATGGCCAATT GGAACCAACA CAATAATGGA GTCCTTGTTG GGAGGTGCGG CAGATTCATC 3840
AATTCGAGAT GGAACGTTGA CATAGAATGA AGCTTCAGAG GGCAAACCGA AGTCGTTGAA 3900
AATCTCATCA AAACTTTCCT TGTAGGCTTC AGCCAAGAAG ATATTGTGTA CGTCTAATTG 3960
AGGCACCTTT GTTGACATGG ACCAATAAAA CGAAATAGAT GATGAAGTGA GTTTCTTTGA 4020
GGCTAATGTC TTCTTTGTCC AATTGCAAGG AGGTAACAGA TGGTGATAAG CATAAACAAG 4080
ATCCGCATTA CATACGACTG CATCGGCTTC AATGACTTCT CCGCTTTCCA AAGTGACACC 4140
GGTTACACGC TTGTCTTTAT CGACAGTGTT AATTTTAGCA ACAGGCGATT GATATCTGAA 4200
TTCAGCACCG TACTTTTTGG AGGCGATAGA CTCAAGCTTC TGAACAACCA TGTTGAAACC 4260
ACCACGAGGA TACCAGATAC CTTCAGCAAA CTCGGTGTAT TGTAACAAAC TGTAAACTGC 4320
TGGAGCATCA TAAGGCGACA TACTATATTC CAAAAATAGA AAATAGAACA ATGAATATCA 4380
AAATTCCTTT CACTTGCCCT TTTTCACATT TCTCTTTTCC CACCCCCGAC CGGTCTCACT 4440
CATTTTTTTT TCATCCCACA CCACGCGTTG TATGTGTACT TACCCCATAT ACATTGTTTG 4500
AAAAGTAAAA GCCATACGCA TTTTCTTGGT TTGGAAATAT TTACTGGCTC GGTCATAGAT 4560
CTTACCAAAC AAGTGCAAGC GAAAGATTTC AGGCACATAC TGAAGACGAA TCAAATCCCA 4620
AATGGTTTCA AAGTTGCGCT TGATAGCAAT AAATGTACCT TGTTCATAAT GGACATGTGT 4680

TTCCTTCATG AAATCCAAGA ATCTACCAAA TCCAAGGGGA CCCTCAATAC GGTCCAATTC 4740
GCCCTTCATC TTGGTTAAAT CGGAAGAGAG TTGTACGGCA TCACCGTCGT CAAAATGAAC 4800
CTTATAGTTA TTGTCACAGC GAAGCAAATC CAAATGATCA CCAATACGTT CATCCAAATC 4860
AGCAAATGCA TCTTCAAAAA GCTTAGGCAT CAAATAGAGT GAGGGACCCT GATCAAAGCG 4920
ATGACCATCG TGATGAATGA ATGAACAACG GCCACCGGAA AAGTCGTTCT TTTCAACAAC 4980
AGTAACTCGA AAACCTTCAC GAGCAAGACG AGCAGCAGTA GCAGTTCCGC CAATACCGGC 5040
ACCAATGACA ACAATATGCT TCTTTTGATC AGACATGAGA TTAAAATAGA TAAGGAAAAG 5100
AAAGTGAAAA GAAATTCGGA AGCATGGCAC ATTCTTCTTT TTATAAATAC ATGCCTGACT 5160
TTCTTTTTCC ATCGATATGA TATATGCATA TGATAGATAT ACAAGCAATC TTCTTCAAGG 5220
AGTTTGAAAT TTTGTCCTCC AGGAGCAAAA AAAAGTTTTT TTTTATACAT GTTTGTACAC 5280
AAGAATAGTT ACCAATTTGC TTTGGTCTTA CGTGCTGCAA GTTTATATCG TTTTCAATTT 5340
CTTTGTCTTT ACATTTTCTT TGTCCTTTAT CTTTCCTCAT TTAGTCTTTG GGAGAATTAG 5400
GAAAAGGGAG CGGAAAGGTA AGAAATGCTT GCGTATTTTA CTAATTCGGC AAACATCCAA 5460
TTTGGCAAAC AGCAGCCTGT GCAACGCTCT CGAGATGACA GTATCTTTGA TTACACTCTA 5520
AATCTCGATG ACCCGACCAA AAAGAGCGAA CAAAGAAATA ATCTTGTGCA TTCGAATATG 5580
ATGGAAGATT TTTTCCCCCT TATTCTAAAT GTTGACATAG CGTGTATGTT ATATAAACAA 5640
AAAGAAATTG TACAAACTTT CTTTTCTTCT CTTTTTATTT TATCTCT ATG TCA ATA 5696
Met Ser Ile
1

CTC ACT TAT CTG GAA TTT CAT CTC TAC TAT ACA CTA CCT GTC CTT GCG 5744
Leu Thr Tyr Leu Glu Phe His Leu Tyr Tyr Thr Leu Pro Val Leu Ala
    5                   10                  15

GCA TTG TGT TGG CTG CTA AAG CCG TTT CAC TCA CAG CAA GAC AAT CTC 5792
Ala Leu Cys Trp Leu Leu Lys Pro Phe His Ser Gln Gln Asp Asn Leu
20                  25                  30                  35

AAG TAT AAA TTT TTA ATG TTG ATG GCC GCC TCT ACC GCA TCG ATT TGG 5840
Lys Tyr Lys Phe Leu Met Leu Met Ala Ala Ser Thr Ala Ser Ile Trp
                40                  45                  50

GAC AAT TAT ATC GTT TAT CAT CGC GCT TGG TGG TAC TGT CCT ACT TGT 5888
Asp Asn Tyr Ile Val Tyr His Arg Ala Trp Trp Tyr Cys Pro Thr Cys
            55                  60                  65

GTT GTG GCT GTC ATT GGC TAT GTA CCT CTA GAA GAA TAC ATG TTC TTT 5936
Val Val Ala Val Ile Gly Tyr Val Pro Leu Glu Glu Tyr Met Phe Phe
        70                  75                  80

ATC ATC ATG ACT TTA ATG ACT GTC GCG TTC TCA AAC TTT GTT ATG CGT 5984
Ile Ile Met Thr Leu Met Thr Val Ala Phe Ser Asn Phe Val Met Arg
    85                  90                  95

TGG CAC TTG CAT ACT TTC TTT ATT AGA CCC AAC ACT TCT TGG AAG CAA 6032
Trp His Leu His Thr Phe Phe Ile Arg Pro Asn Thr Ser Trp Lys Gln
100                 105                 110                 115

ACA CTA TTA GTA CGC CTT GTG CCT GTT TCA GCT TTA TTG GCA ATC ACT 6080
Thr Leu Leu Val Arg Leu Val Pro Val Ser Ala Leu Leu Ala Ile Thr
                120                 125                 130

TAT CAT GCT TGG GTATGCAAAA TAAACAAACA CTAAAAAAAA ATAATAGCGA 6132
Tyr His Ala Trp
            135

TAATTATTTT ACTCATTTTT CTTTTTTTAG CAC TTG ACA CTG CCA AAT AAA TCT 6186
His Leu Thr Leu Pro Asn Lys Ser
                140

TCA TTT TAT GGT TCA TGC ATC CTT TGG TAT GCT TGT CCT GTG TTG GCT 6234
Ser Phe Tyr Gly Ser Cys Ile Leu Trp Tyr Ala Cys Pro Val Leu Ala
    145                 150                 155

ATT CTT TGG CTG GGT GCT GGT GAA TAT ATC TTG CGT CGA CCT GTG GCT 6282
Ile Leu Trp Leu Gly Ala Gly Glu Tyr Ile Leu Arg Arg Pro Val Ala
160                 165                 170                 175

GTC CTT TTG TCT ATT GTT ATC CCT AGT GTA TAC CTA TGT TGG GCT GAT 6330
Val Leu Leu Ser Ile Val Ile Pro Ser Val Tyr Leu Cys Trp Ala Asp
                180                 185                 190

ATC GTC GCT ATT AGT GCT GGC ACA TGG CAT ATT TCT CTT AGA ACA AGC 6378
Ile Val Ala Ile Ser Ala Gly Thr Trp His Ile Ser Leu Arg Thr Ser
            195                 200                 205

ACT GGC AAA ATG GTA GTA CCC GAT TTA CCT GTA GAA GAA TGC CTG TTT 6426
Thr Gly Lys Met Val Val Pro Asp Leu Pro Val Glu Glu Cys Leu Phe
        210                 215                 220

TTT ACT TTG ATC AAC ACA GTC TTG GTT TTT GCT ACC TGT GCT ATA GAC 6474
Phe Thr Leu Ile Asn Thr Val Leu Val Phe Ala Thr Cys Ala Ile Asp
    225                 230                 235

CGC GCT CAG GCC ATC CTC CAT CTG TAC AAA TCA TCT GTT CAA AAT CAA 6522
Arg Ala Gln Ala Ile Leu His Leu Tyr Lys Ser Ser Val Gln Asn Gln
240                 245                 250                 255

AAC CCT AAA CAA GCC ATT TCC CTT TTC CAG CAT GTC AAA GAG CTA GCA 6570
Asn Pro Lys Gln Ala Ile Ser Leu Phe Gln His Val Lys Glu Leu Ala
                260                 265                 270

TGG GCC TTC TGT CTT CCT GAC CAA ATG CTC AAC AAT GAA TTG TTT GAT 6618
Trp Ala Phe Cys Leu Pro Asp Gln Met Leu Asn Asn Glu Leu Phe Asp
            275                 280                 285

GAT CTT ACT ATC AGC TGG GAT ATT TTA CGT AAA GCC TCA AAG TCA TTC 6666
Asp Leu Thr Ile Ser Trp Asp Ile Leu Arg Lys Ala Ser Lys Ser Phe
        290                 295                 300

TAT ACT GCA TCT GCC GTT TTT CCA AGT TAT GTA CGT CAA GAC TTG GGT 6714
Tyr Thr Ala Ser Ala Val Phe Pro Ser Tyr Val Arg Gln Asp Leu Gly
    305                 310                 315

GTT CTC TAT GCT TTC TGC AGA GCT ACC GAT GAC CTG TGC GAT GAT GAA 6762
Val Leu Tyr Ala Phe Cys Arg Ala Thr Asp Asp Leu Cys Asp Asp Glu
320                 325                 330                 335

TCC AAA TCT GTT CAA GAA AGA AGA GAC CAA TTA GAT CTT ACT CGA CAA 6810
Ser Lys Ser Val Gln Glu Arg Arg Asp Gln Leu Asp Leu Thr Arg Gln
                340                 345                 350

TTT GTT CGT GAT CTC TTT AGC CAA AAG ACC AGT GCG CCT ATT GTG ATT 6858
Phe Val Arg Asp Leu Phe Ser Gln Lys Thr Ser Ala Pro Ile Val Ile
            355                 360                 365

GAT TGG GAA TTG TAT CAA AAC CAA CTT CCT GCT TCT TGT ATA TCA GCC 6906
Asp Trp Glu Leu Tyr Gln Asn Gln Leu Pro Ala Ser Cys Ile Ser Ala
        370                 375                 380

TTT AGA GCC TTT ACT CGC CTT CGC CAT GTC CTT GAA GTA GAC CCT GTA 6954
Phe Arg Ala Phe Thr Arg Leu Arg His Val Leu Glu Val Asp Pro Val
    385                 390                 395

GAA GAA CTA TTA GAT GGT TAC AAA TGG GAT CTT GAG CGT CGT CCT ATC 7002
Glu Glu Leu Leu Asp Gly Tyr Lys Trp Asp Leu Glu Arg Arg Pro Ile
400                 405                 410                 415

CTT GAT GAA CAA GAC TTG GAG GCA TAC TCT GCT TGT GTG GCC AGT AGT 7050
Leu Asp Glu Gln Asp Leu Glu Ala Tyr Ser Ala Cys Val Ala Ser Ser
                420                 425                 430

GTG GGT GAA ATG TGC ACA CGT GTG ATT CTT GCT CAA GAC CAA AAG GAA 7098
Val Gly Glu Met Cys Thr Arg Val Ile Leu Ala Gln Asp Gln Lys Glu
            435                 440                 445

AAT GAT GCT TGG ATA ATT GAC CGT GCA CGT GAG ATG GGG CTG GTG CTA 7146
Asn Asp Ala Trp Ile Ile Asp Arg Ala Arg Glu Met Gly Leu Val Leu
        450                 455                 460

CAA TAC GTT AAC ATT GCT CGA GAC ATT GTG ACT GAT AGC GAG ACT CTG 7194
Gln Tyr Val Asn Ile Ala Arg Asp Ile Val Thr Asp Ser Glu Thr Leu
    465                 470                 475

GGT CGA TGT TAT CTG CCT CAA CAA TGG CTT AGA AAA GAA GAA ACA GAA 7242
Gly Arg Cys Tyr Leu Pro Gln Gln Trp Leu Arg Lys Glu Glu Thr Glu
480                 485                 490                 495

CAA ATA CAG CAA GGC AAC GCC CGT AGC CTA GGT GAT CAA AGA CTG TTG 7290
Gln Ile Gln Gln Gly Asn Ala Arg Ser Leu Gly Asp Gln Arg Leu Leu
                500                 505                 510

GGC TTG TCT CTG AAG CTT GTA GGA AAG GCA GAC GCT ATC ATG GTG AGA 7338
Gly Leu Ser Leu Lys Leu Val Gly Lys Ala Asp Ala Ile Met Val Arg
            515                 520                 525

GCT AAG AAG GGC ATT GAC AAG TTG CCG GCA AAC TGT CAA GGC GGT GTA 7386
Ala Lys Lys Gly Ile Asp Lys Leu Pro Ala Asn Cys Gln Gly Gly Val
        530                 535                 540

CGA GCT GCT TGC CAA GTA TAT GCT GCA ATT GGA TCT GTA CTC AAG CAG 7434
Arg Ala Ala Cys Gln Val Tyr Ala Ala Ile Gly Ser Val Leu Lys Gln
    545                 550                 555

CAG AAG ACA ACA TAT CCT ACA AGA GCT CAT CTA AAA GGA AGC GAA CGT 7482
Gln Lys Thr Thr Tyr Pro Thr Arg Ala His Leu Lys Gly Ser Glu Arg
560                 565                 570                 575

GCC AAG ATT GCT CTG TTG AGT GTA TAC AAC CTC TAT CAA TCT GAA GAC 7530
Ala Lys Ile Ala Leu Leu Ser Val Tyr Asn Leu Tyr Gln Ser Glu Asp
                580                 585                 590

AAG CCT GTG GCT CTC CGT CAA GCT AGA AAG ATT AAG AGT TTT TTT GTT 7578
Lys Pro Val Ala Leu Arg Gln Ala Arg Lys Ile Lys Ser Phe Phe Val
            595                 600                 605

GAT TAG TGAATTTTTG TTTTATTTAT GTCTGATAGT TCAATAAAGA GACAACACAT 7634
Asp

ACAATATAAA ATCATTGTCT TTAAATGTTA ATTTAGTAGA GTGTAAAGCC TGCATTTTTT 7694
TTGTACGCAT AAACAATGAG TTCACCCCGC TTCTGGTTTT TAAATAATTA TGTCAAACTA 7754
GGGAAAATTC TTTTTTTTCT CTTCGTTCTT TTTTTGGCTT GTTGTGGAGT CACAGGCTTG 7814
TCTTCAGATT GATAGAGGTT GTATACACTC AACAGAGCAA TCTTGGCACG TTCGCTTCCT 7874
TTTAGATGAG CTCTTGTAGG ATATGTTGTC TTCTGCTGCT TGAGTACAGA TCCAATTGCA 7934
GCATATACTT GGCAAGCAGC TCGTACACCG CCTTGACAGT TTGCCGGCAA CTTGTCAATG 7994
CCCTTCTTAG CTCTCACCAT GATAGCGTCT GCCTTTCCTA CAAGCTTCAG AGACAAGCCC 8054
AACAGTCTTT GATCACCTAG GCTACGGGCG TTGCCTTGCT GTATTTGTTC TGTTTCTTCT 8114
TTTCTAAGCC ATTGTTGAGG CAGATAACAT CGACCCAACA TCCTCGAGCC ATACTACAGC 8174
ATAAAAGGAT ACGTTTTCTT TAACAGAAAT TTACCCTTTT GTTATCAGCA CATACAAAAA 8234
AAAAGAAATT TAAGATGAGT AGGACTTCCA TTCTCTCAAA AATTTTATTC AATCCATAAA 8294
TGAATTATTT TTGGACAAAA AAGAAAGATT ATGCCTGATT TTCTCTATTT TTTTTTTTTT 8354
TACAACTCCA CCAATACTTT CTAGAGACAC ATTTGAGCGA TGTGACAGTC GGACTCGAGA 8414
AGTACAAGAA GGTACAGAAA TAGCAGCTGA GCGTATGATT GGGTCCCAAG GTTCTTCTCG 8474
ACTTTCTCTC TTACCCTGGA ACAGAGAGAA AAAAAAAATA TTTCGTCTTT TTTGGATAAT 8434
ATTATAAAAA AGGGAATTTA GTAAAGAAAA CGGTTGTTTC CTTTTCTTTT TTTTTTTCCT 8494
TCTCCACTAC ATGAATAAAC ATCGCCACCC AAATTTACCT TCCATATCTA CTCTACTTAC 8554
TGGACCACCT TCTCCGCCAC CTCCCATTAT TGTGATAGAT GAGGAACATT CTCCCAGTTG 8614
TTCACCTAAC AAATACCACT TGTCTCCTGT CTTATCACCT ATTGATTCAT ATGCTTCATC 8674
ACCAAACTCG AG 8786

<210> SEQ ID NO: 2

<211> 8786

<212> DNA

<213> Blakeslea trispora

<220> CDS

<221> carB 3711...5665

<220> Exon 3711...4303

<220> Exon 4445...5293

<220> Exon 5362...5665

<223> Immediate source: plasmids pALBT9 (CECT 5982) and
pALBT52 (CECT 5981).

<223> β-Carotene biosynthesis genes.

<400>

CTCGAGTTTG GTGATGAAGC ATATGAATCA ATAGGTGATA AGACAGGAGA CAAGTGGTAT 60
TTGTTAGGTG AACAACTGGG AGAATGTTCC TCATCTATCA CAATAATGGG AGGTGGCGGA 120
GAAGGTGGTC CAGTAAGTAG AGTAGATATG GAAGGTAAAT TTGGGTGGCG ATGTTTATTC 180
ATGTAGTGGA GAAGGAAAAA AAAAAAGAAA AGGAAACAAC CGTTTTCTTT ACTAAATTCC 240
CTTTTTTATA ATATTATCCA AAAAAGACGA AATATTTTTT TTTTCTCTCT GTTCCAGGGT 300
AAGAGAGAAA GTCGAGAAGA ACCTTGGGAC CCAATCATAC GCTCAGCTGC TATTTCTGTA 360
CCTTCTTGTA CTTCTCGAGT CCGACTGTCA CATCGCTCAA ATGTGTCTCT AGAAAGTATT 420
GGTGGAGTTG TAAAAAAAAA AAAAATAGAG AAAATCAGGC ATAATCTTTC TTTTTTGTCC 480
AAAAATAATT CATTTATGGA TTGAATAAAA TTTTTGAGAG AATGGAAGTC CTACTCATCT 540
TAAATTTCTT TTTTTTTGTA TGTGCTGATA ACAAAAGGGT AAATTTCTGT TAAAGAAAAC 600
GTATCCTTTT ATGCTGTAGT ATGGCTCGAG GATGTTGGGT CGATGTTATC TGCCTCAACA 660
ATGGCTTAGA AAAGAAGAAA CAGAACAAAT ACAGCAAGGC AACGCCCGTA GCCTAGGTGA 720
TCAAAGACTG TTGGGCTTGT CTCTGAAGCT TGTAGGAAAG GCAGACGCTA TCATGGTGAG 780
AGCTAAGAAG GGCATTGACA AGTTGCCGGC AAACTGTCAA GGCGGTGTAC GAGCTGCTTG 840
CCAAGTATAT GCTGCAATTG GATCTGTACT CAAGCAGCAG AAGACAACAT ATCCTACAAG 900
AGCTCATCTA AAAGGAAGCG AACGTGCCAA GATTGCTCTG TTGAGTGTAT ACAACCTCTA 960
TCAATCTGAA GACAAGCCTG TGACTCCACA ACAAGCCAAA AAAAGAACGA AGAGAAAAAA 1020
AAGAATTTTC CCTAGTTTGA CATAATTATT TAAAAACCAG AAGCGGGGTG AACTCATTGT 1080
TTATGCGTAC AAAAAAAATG CAGGCTTTAC ACTCTACTAA ATTAACATTT AAAGACAATG 1140
ATTTTATATT GTATGTGTTG TCTCTTTATT GAACTATCAG ACATAAATAA AACAAAAATT 1200
CACTAATCAA CAAAAAAACT CTTAATCTTT CTAGCTTGAC GGAGAGCCAC AGGCTTGTCT 1260
TCAGATTGAT AGAGGTTGTA TACACTCAAC AGAGCAATCT TGGCACGTTC GCTTCCTTTT 1320
AGATGAGCTC TTGTAGGATA TGTTGTCTTC TGCTGCTTGA GTACAGATCC AATTGCAGCA 1380
TATACTTGGC AAGCAGCTCG TACACCGCCT TGACAGTTTG CCGGCAACTT GTCAATGCCC 1440
TTCTTAGCTC TCACCATGAT AGCGTCTGCC TTTCCTACAA GCTTCAGAGA CAAGCCCAAC 1500
AGTCTTTGAT CACCTAGGCT ACGGGCGTTG CCTTGCTGTA TTTGTTCTGT TTCTTCTTTT 1560
CTAAGCCATT GTTGAGGCAG ATAACATCGA CCCAGAGTCT CGCTATCAGT CACAATGTCT 1620
CGAGCAATGT TAACGTATTG TAGCACCAGC CCCATCTCAC GTGCACGGTC AATTATCCAA 1680
GCATCATTTT CCTTTTGGTC TTGAGCAAGA ATCACACGTG TGCACATTTC ACCCACACTA 1740
CTGGCCACAC AAGCAGAGTA TGCCTCCAAG TCTTGTTCAT CAAGGATAGG ACGACGCTCA 1800
AGATCCCATT TGTAACCATC TAATAGTTCT TCTACAGGGT CTACTTCAAG GACATGGCGA 1860
AGGCGAGTAA AGGCTCTAAA GGCTGATATA CAAGAAGCAG GAAGTTGGTT TTGATACAAT 1920
TCCCAATCAA TCACAATAGG CGCACTGGTC TTTTGGCTAA AGAGATCACG AACAAATTGT 1980

CGAGTAAGAT CTAATTGGTC TCTTCTTTCT TGAACAGATT TGGATTCATC ATCGCACAGG 2040
TCATCGGTAG CTCTGCAGAA AGCATAGAGA ACACCCAAGT CTTGACGTAC ATAACTTGGA 2100
AAAACGGCAG ATGCAGTATA GAATGACTTT GAGGCTTTAC GTAAAATATC CCAGCTGATA 2160
GTAAGATCAT CAAACAATTC ATTGTTGAGC ATTTGGTCAG GAAGACAGAA GGCCCATGCT 2220
AGCTCTTTGA CATGCTGGAA AAGGGAAATG GCTTGTTTAG GGTTTTGATT TTGAACAGAT 2280
GATTTGTACA GATGGAGGAT GGCCTGAGCG CGGTCTATAG CACAGGTAGC AAAAACCAAG 2340
ACTGTGTTGA TCAAAGTAAA AAACAGGCAT TCTTCTACAG GTAAATCGGG TACTACCATT 2400
TTGCCAGTGC TTGTTCTAAG AGAAATATGC CATGTGCCAG CACTAATAGC GACGATATCA 2460
GCCCAACATA GGTATACACT AGGGATAACA ATAGACAAAA GGACAGCCAC AGGTCGACGC 2520
AAGATATATT CACCAGCACC CAGCCAAAGA ATAGCCAACA CAGGACAAGC ATACCAAAGG 2580
ATGCATGAAC CATAAAATGA AGATTTATTT GGCAGTGTCA AGTGCTAAAA AAAGAAAAAT 2640
GAGTAAAATA ATTATCGCTA TTATTTTTTT TTAGTGTTTG TTTATTTTGC ATACCCAAGC 2700
ATGATAAGTG ATTGCCAATA AAGCTGAAAC AGGCACAAGG CGTACTAATA GTGTTTGCTT 2760
CCAAGAAGTG TTGGGTCTAA TAAAGAAAGT ATGCAAGTGC CAACGCATAA CAAAGTTTGA 2820
GAACGCGACA GTCATTAAAG TCATGATGAT AAAGAACATG TATTCTTCTA GAGGTACATA 2880
GCCAATGACA GCCACAACAC AAGTAGGACA GTACCACCAA GCGCGATGAT AAACGATATA 2940
ATTGTCCCAA ATCGATGCGG TAGAGGCGGC CATCAACATT AAAAATTTAT ACTTGAGATT 3000
GTCTTGCTGT GAGTGAAACG GCTTTAGCAG CCAACACAAT GCCGCAAGGA CAGGTAGTGT 3060
ATAGTAGAGA TGAAATTCCA GATAAGTGAG TATTGACATA GAGATAAAAT AAAAAGAGAA 3120
GAAAAGAAAG TTTGTACAAT TTCTTTTTGT TTATATAACA TACACGCTAT GTCAACATTT 3180
AGAATAAGGG GGAAAAAATC TTCCATCATA TTCGAATGCA CAAGATTATT TCTTTGTTCG 3240
CTCTTTTTGG TCGGGTCATC GAGATTTAGA GTGTAATCAA AGATACTGTC ATCTCGAGAG 3300
CGTTGCACAG GCTGCTGTTT GCCAAATTGG ATGTTTGCCG AATTAGTAAA ATACGCAAGC 3360
ATTTCTTACC TTTCCGCTCC CTTTTCCTAA TTCTCCCAAA GACTAAATGA GGAAAGATAA 3420
AGGACAAAGA AAATGTAAAG ACAAAGAAAT TGAAAACGAT ATAAACTTGC AGCACGTAAG 3480
ACCAAAGCAA ATTGGTAACT ATTCTTGTGT ACAAACATGT ATAAAAAAAA ACTTTTTTTT 3540
GCTCCTGGAG GACAAAATTT CAAACTCCTT GAAGAAGATT GCTTGTATAT CTATCATATG 3600
CATATATCAT ATCGATGGAA AAAGAAAGTC AGGCATGTAT TTATAAAAAG AAGAATGTGC 3660
CATGCTTCCG AATTTCTTTT CACTTTCTTT TCCTTATCTA TTTTAATCTC ATG TCT 3716
Met Ser
1

GAT CAA AAG AAG CAT ATT GTT GTC ATT GGT GCC GGT ATT GGC GGA ACT 3764
Asp Gln Lys Lys His Ile Val Val Ile Gly Ala Gly Ile Gly Gly Thr
        5                   10                  15

GCT ACT GCT GCT CGT CTT GCT CGT GAA GGT TTT CGA GTT ACT GTT GTT 3812
Ala Thr Ala Ala Arg Leu Ala Arg Glu Gly Phe Arg Val Thr Val Val
    20                  25                  30

GAA AAG AAC GAC TTT TCC GGT GGC CGT TGT TCA TTC ATT CAT CAC GAT 3860
Glu Lys Asn Asp Phe Ser Gly Gly Arg Cys Ser Phe Ile His His Asp
35                  40                  45                  50

GGT CAT CGC TTT GAT CAG GGT CCC TCA CTC TAT TTG ATG CCT AAG CTT 3908
Gly His Arg Phe Asp Gln Gly Pro Ser Leu Tyr Leu Met Pro Lys Leu
                55                  60                  65

TTT GAA GAT GCA TTT GCT GAT TTG GAT GAA CGT ATT GGT GAT CAT TTG 3956
Phe Glu Asp Ala Phe Ala Asp Leu Asp Glu Arg Ile Gly Asp His Leu
            70                  75                  80

GAT TTG CTT CGC TGT GAC AAT AAC TAT AAG GTT CAT TTT GAC GAC GGT 4004
Asp Leu Leu Arg Cys Asp Asn Asn Tyr Lys Val His Phe Asp Asp Gly
        85                  90                  95

GAT GCC GTA CAA CTC TCT TCC GAT TTA ACC AAG ATG AAG GGC GAA TTG 4052
Asp Ala Val Gln Leu Ser Ser Asp Leu Thr Lys Met Lys Gly Glu Leu
    100                 105                 110

GAC CGT ATT GAG GGT CCC CTT GGA TTT GGT AGA TTC TTG GAT TTC ATG 4100
Asp Arg Ile Glu Gly Pro Leu Gly Phe Gly Arg Phe Leu Asp Phe Met
115                 120                 125                 130

AAG GAA ACA CAT GTC CAT TAT GAA CAA GGT ACA TTT ATT GCT ATC AAG 4148
Lys Glu Thr His Val His Tyr Glu Gln Gly Thr Phe Ile Ala Ile Lys
                135                 140                 145

CGC AAC TTT GAA ACC ATT TGG GAT TTG ATT CGT CTT CAG TAT GTG CCT 4196
Arg Asn Phe Glu Thr Ile Trp Asp Leu Ile Arg Leu Gln Tyr Val Pro
            150                 155                 160

GAA ATC TTT CGC TTG CAC TTG TTT GGT AAG ATC TAT GAC CGA GCC AGT 4244
Glu Ile Phe Arg Leu His Leu Phe Gly Lys Ile Tyr Asp Arg Ala Ser
        165                 170                 175

AAA TAT TTC CAA ACC AAG AAA ATG CGT ATG GCT TTT ACT TTT CAA ACA 4292
Lys Tyr Phe Gln Thr Lys Lys Met Arg Met Ala Phe Thr Phe Gln Thr
    180                 185                 190

ATG TAT ATG GG GTAAGTACAC ATACAACGCG TGGTGTGGGA TGAAAAAAA 4343
Met Tyr Met Gly
195

ATGAGTGAGA CCGGTCGGGG GTGGGAAAAG AGAAATGTGA AAAAGGGCAA GTGAAAGGAA 4403
TTTTGATATT CATTGTTCTA TTTTCTATTT TTGGAATATA G T ATG TCG CCT TAT 4457
Met Ser Pro Tyr
    200

GAT GCT CCA GCA GTT TAC AGT TTG TTA CAA TAC ACC GAG TTT GCT GAA 4505
Asp Ala Pro Ala Val Tyr Ser Leu Leu Gln Tyr Thr Glu Phe Ala Glu
        205                 210                 215

GGT ATC TGG TAT CCT CGT GGT GGT TTC AAC ATG GTT GTT CAG AAG CTT 4553
Gly Ile Trp Tyr Pro Arg Gly Gly Phe Asn Met Val Val Gln Lys Leu
    220                 225                 230

GAG TCT ATC GCC TCC AAA AAG TAC GGT GCT GAA TTC AGA TAT CAA TCG 4601
Glu Ser Ile Ala Ser Lys Lys Tyr Gly Ala Glu Phe Arg Tyr Gln Ser
235                 240                 245                 250

CCT GTT GCT AAA ATT AAC ACT GTC GAT AAA GAC AAG CGT GTA ACC GGT 4649
Pro Val Ala Lys Ile Asn Thr Val Asp Lys Asp Lys Arg Val Thr Gly
                255                 260                 265

GTC ACT TTG GAA AGC GGA GAA GTC ATT GAA GCC GAT GCA GTC GTA TGT 4697
Val Thr Leu Glu Ser Gly Glu Val Ile Glu Ala Asp Ala Val Val Cys
            270                 275                 280

AAT GCG GAT CTT GTT TAT GCT TAT CAC CAT CTG TTA CCT CCT TGC AAT 4745
Asn Ala Asp Leu Val Tyr Ala Tyr His His Leu Leu Pro Pro Cys Asn
        285                 290                 295

Tì¥Á[[ [[[ð[¿[[[[[[[[[[[[[[[Ï±[[

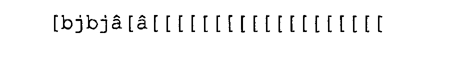

[[[[[[[[[[â[[[[[[[ E[[[[[[ E[[[[[[ E[[8[[[XE[[$[ì¥Á[[
[[[õ[¿[[[[[[[[[[[[[[[Ï±[[

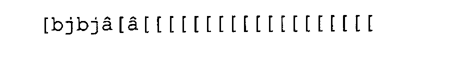

E[[[[[[ E[[8[[[XE[[$[lu Ala Tyr Lys Glu Ser Phe Asp Glu Ile Phe
                    335                 340                 345

AAC GAC TTC GGT TTG CCC TCT GAA GCT TCA TTC TAT GTC AAC GTT CCA   4937
Asn Asp Phe Gly Leu Pro Ser Glu Ala Ser Phe Tyr Val Asn Val Pro
            350                 355                 360

TCT CGA ATT GAT GAA TCT GCC GCA CCT CCC AAC AAG GAC TCC ATT ATT   4985
Ser Arg Ile Asp Glu Ser Ala Ala Pro Pro Asn Lys Asp Ser Ile Ile
        365                 370                 375

GTG TTG GTT CCA ATT GGC CAT ATG AAG AGT AAG ACA GGA AAC AGT GCT   5033
Val Leu Val Pro Ile Gly His Met Lys Ser Lys Thr Gly Asn Ser Ala
    380                 385                 390

GAA GAA AAT TAT CCT GAG TTG GTA AAC CGT GCA CGC AAG ATG GTT CTG 5081
Glu Glu Asn Tyr Pro Glu Leu Val Asn Arg Ala Arg Lys Met Val Leu
395 400 405 410

GAA GTT ATC GAA CGT CGT TTG GGA GTA AAC AAC TTT GCT AAT TTG ATT 5129
Glu Val Ile Glu Arg Arg Leu Gly Val Asn Asn Phe Ala Asn Leu Ile
415 420 425

GAA CAT GAA GAA GTG AAT GAT CCT AGT GTT TGG CAA AGC AAG TTT AAC 5177
Glu His Glu Glu Val Asn Asp Pro Ser Val Trp Gln Ser Lys Phe Asn
430 435 440

CTT TGG AGA GGT TCT ATT CTT GGT CTT TCT CAT GAT GTG TTC CAA GTT 5225
Leu Trp Arg Gly Ser Ile Leu Gly Leu Ser His Asp Val Phe Gln Val
445 450 455

CTC TGG TTC AGA CCT AGT ACC AAG GAT TCC ACA AAC CGT TAT GAT AAT 5273
Leu Trp Phe Arg Pro Ser Thr Lys Asp Ser Thr Asn Arg Tyr Asp Asn
460 465 470

CTT TTC TTT GTC GGA GCT AG GTAATACTTT TGCCCTCTTT TTTTCTTTTT 5323
Leu Phe Phe Val Gly Ala Ser
475 480

TTTAATAACG CACGTACTCA TTTCTCTTTT CTTTGTAG T ACA CAT CCA GGT ACT 5377
Thr His Pro Gly Thr
485

GGT GTT CCT ATC GTT CTT GCT GGA AGT AAG CTT ACT TCC GAC CAA GTC 5425
Gly Val Pro Ile Val Leu Ala Gly Ser Lys Leu Thr Ser Asp Gln Val
490 495 500

TGT AAA AGC TTT GGC CAG AAT CCC TTA CCA AGA AAG TTA CAA GAT AGC 5473
Cys Lys Ser Phe Gly Gln Asn Pro Leu Pro Arg Lys Leu Gln Asp Ser
505 510 515

CAA AAG AAG TAT GCT CCT GAA CAA ACT CGT AAG ACC GAA AGC CAT TGG 5521
Gln Lys Lys Tyr Ala Pro Glu Gln Thr Arg Lys Thr Glu Ser His Trp
520 525 530

ATC TAT TAT TGT CTT GCT TGT TAC TTT GTT ACT TTC CTC TTT TTC TAT 5569
Ile Tyr Tyr Cys Leu Ala Cys Tyr Phe Val Thr Phe Leu Phe Phe Tyr
535 540 545 550

TTC TTC CCA AGA GAT GAT ACT ACA ACT CCT GCT TCT TTC ATT AAC CAA 5617
Phe Phe Pro Arg Asp Asp Thr Thr Thr Pro Ala Ser Phe Ile Asn Gln
555 560 565

CTT TTA CCT AAC GTT TTC CAA GGA CAA AAC AGC AAC GAT ATT CGC ATT 5665
Leu Leu Pro Asn Val Phe Gln Gly Gln Asn Ser Asn Asp Ile Arg Ile
570 575 580

TAGT TCAATAATAA AGCTACCATT TTAACATCAT ATGAACTTAA CCATTTAAAT 5719
GTATCACTCT GTGTCTGCGT TCTCTTTGAT CATAGAATTC TTTCTTTTTT TGTGCGTATA 5779
TTTGTATCCA TTATCTAGCA CGTCATTAAA TTGGTGTATG TTGATCCTTT CGAATGGAAT 5839
GAATTCTAGA ATAGTGTCTA AAAAGACAAT TCACATGTGT ATCAGAATCA GCTCAAACAA 5899
AGAAACCCAA AATAAATGAC TCGACGCAAA TAGAAAACTG AATTGTTTGG TAGGACAGAC 5959
AATTCTACAA ACAAATATTG GAAAAACAAA TTATCCTGAT TTTAGAAAAA ATAATCGAAT 6019
TTAGTTTTAT TATGATATGC ACAAATTTTG TTTCTACTAA ATGTGGTATT TGATTGAGAG 6079
TAACCATTAA AAATCAATCC TAGCAAACAT TTCTTTTTAA AAATAAGGCA AAGGAGCCAT 6139
ACATAGATCA AGCACAGCAT TATCCAAAAA AGCAAAACAC GTCTGTAAGG TAGGTGAGTG 6199
ATACGAGTCT TGCGTAAAAA AAGAAAATAA TCATTAGCTT GGGAGGGTTT TCAAGACAAT 6259
GAAATGGTAA AAAAAAAAAC GGTATTCTAA AAGGACTAAC CTTAAAACGA AAATAGAGAT 6319
TTTTCGTTTG AAACGTCATA ATCATAACAT TCACCGGAGT TGCTGGTGTG TTTCTTTTAC 6379
TAAACCAAAG AGTAAAGCAT CCTCACTTGA TCTGATTAAA CATGTCAAAC AAAACATTTC 6439
AACAGACACA ATGTCTCTTT GTTGATATTG CAAAGAAGCA TAAACAGTGG CTTTGTGTAG 6499
TACCTAAATT ACTATTCTCT TGAAAGCCAT AGCTTTGATA AGCTTTTTTC TCCCTTTGGC 6559
TTGATTGCTT ATTGCCACAT CTAGAAAAGT GGTTTTACTT TGCCTGATAG AAAATAAAAA 6619
CGTTCTTTTC ATTGTGCTTA TCTTAACTTT AGACTTATTT TTAACCCATC TCAATTGCAA 6679

CAAGCGATCT ACAAGACCTT TTTGGTAAAG GTTCAGTCTT CAAATAACAT GATTACTCAG 6739
TGTTTATAAC ACCAAATTAA TGCGGATGCT TCCTCAAAAA TACATTCAAG TGTAACATAC 6799
TCCTCTTCCT CTCCCTTTTT ATTCGGGCAT GCAGTAAAAG AATCTCGGTG GATTTGCCCT 6859
TTGTCAAGCA ATATAGCTTT TCTTTGTAAG ATTGTTTCAA GAAAAACAGA GATTTAGCTC 6919
ATGATAGCTA GCAAATTCTT TTTCTTTTCT TTGAGCTTTG AATTAAGACT GCACCACATC 6979
GTTAAAGCAA AATTGTTATC ATTCTAGACT GCAATATATT TTCATTGTAC TTTAACTATT 7039
AGTTAAAGTT TTGAGCAACA GTATTGTTGT GTGTATGTGT CACATTCTAC GGTGTTTGTC 7099
AGTTTTTTTT AAGGAAAGTC GTCTACGGCG ATAAGCATCA GAGTTGCACA TGGATCGCTT 7159
TCTCTGTTAT AGCAAGTACT ATTTAGCGAT ATATGTCTTC TGTTTGATAT GATGATATTC 7219
AAGCTATCAG CTTTAACGAA AAAGAAATTG TGTGAGTAAA ATTGTCTATC TTCAAAGCAA 7279
GGAAGATGGA AAACTACAAA AAAAAAAGAA GTAGAGTATA GTAAAACTAA GCTATTCTGT 7339
CGCTTACATC TTTCTTGTCC TTCCTACACG TGTTGTTGAA AACAAAGAAA TAGTCAGAGG 7399
ACTAGAAGCA AGATGATAGT AATGGTATTT TTAACCTAGT CTTTGAAATC TTTCAAAAAA 7459
GATGGCCATG ATGTTTATTA AGGAAAGTTT TGTAGGCTTT TCCAATACGT TTTTCTTCAC 7519
ATTTAGCCAT CTATTGAGTC ATTCCATTGG CAATAAGCCT GTACTACATT TGTCAGAGTG 7579
CAAGAGAGGG AGTTATCTGA GGCTTTATAC CGCCCTTTTT TTTGTCATGG CTCTGTCCAT 7639
GCTTTAGAAA GCCATATACC AAACCTCAAA GACTATCACT TTATTTCAAT TGTACTTAAA 7699
CAGATCACTT CCCTGAAGCT CATATTCACA AAAACAAAAG TGTATCAGGC AAAATCAATA 7759
CAATCTCAAG ATACTATCCT AAAGATGAGA TTCGAAAATC AAAACGCAGA TAGAAAACAT 7819
ACAAACACAA TGCTACCGTG CCATTCACTT ATTACTGATG GGTTGAGGAA TAGCTACTCT 7879
TGCATTATGC TGAATAGCCA AAAAAAGAAA GAGGCAAGTG GAAAGAGAAA AAGACATCTA 7939
TTTTGGAATG GAAGCAACAC AATTCATGAT GCTAAAGCAT GTAAATCAAT GAGCAGAGTA 7999
GCCCTTTATA AATCATCTGC TTGCTTAATC TGTCTCCTAG TTTGATGTTG CTTGTCATTG 8059
AATGTTTGTA TGACATTGCT CAATAATGAT CGAACAGAAG GAGCAAATGC ACAAAGCGAG 8119
TCCAAGTCTC AATCCACCCC ATTTTTGGTT TCTCAAAGGA TTAATAAGAG TCAGATAAAG 8179
CATTCTCAGA CAATAAAGAC AAGGAGATGG TTGAGTTCCT GAAAAGAAGA GACAGGATAA 8239
AATAGCCATA CAATTCTGAC TTTTGTATTA TGTAAGCGGT CTTTATTTTT TCAGAAAAAA 8299
AATCATGCTT TAAAATTAAC ATTGTCAAGA GAAAACAGCT ACAAATCGTT CAAGTTAAAA 8359
AAGATAGATG TCATATTCAA TACAGCAATT ATAAAAGTGC CTAAATGTAA AGCTTAAAAG 8419
AAGCTCAAGC AAAAAAAAAA GACTGTTGAT CGCCTAAAAG TAAGTCTCTC TTTTTTTTTA 8479
TTTTCTTTCT TTTAGAAGAA ATTTCGGGCT TTTTGGGTTT CTGCAAATAA CTTTTTATTT 8539
TTTATTTTTT ATTTTATTCA TTTGGCTTTC AGTGTACAAG GTCCCTTTTT ATTTTATTTT 8599
GTTATATCAA GGCATGAACA GTCCAAAAAG TATATTGAAG TTTAGGCCAG AAATAAATAC 8659
CACTGTATCA AAGCCTGCTG TTGTATCACC CACAAGGCCT ACAAGTGGCT CCAACTGGCT 8719
CAGTCGTATA CAATCCAAGC TTTATTCTCA ACAATTTATT AACAACAGTG ATGATACAAG 8779
TGAGCCC 8786

<210> SEQ ID NO:3
<211> 608
<212> polypeptide
<213> deduced
<223> Pm 69581Da
<223> Type of sequence: deduced amino acid sequence encoded
by the carRP gene of B. trispora.

<400>

Met Ser Ile Leu Thr Tyr Leu Glu Phe His Leu Tyr Tyr Thr
1               5                   10
Leu Pro Val Leu Ala Ala Leu Cys Trp Leu Leu Lys Pro Phe His Ser
15                  20                  25                  30
Gln Gln Asp Asn Leu Lys Tyr Lys Phe Leu Met Leu Met Ala Ala Ser
                35                  40                  45
Thr Ala Ser Ile Trp Asp Asn Tyr Ile Val Tyr His Arg Ala Trp Trp
            50                  55                  60
Tyr Cys Pro Thr Cys Val Val Ala Val Ile Gly Tyr Val Pro Leu Glu
        65                  70                  75

Glu Tyr Met Phe Phe Ile Ile Met Thr Leu Met Thr Val Ala Phe Ser
    80                  85                  90
Asn Phe Val Met Arg Trp His Leu His Thr Phe Phe Ile Arg Pro Asn
95                  100                 105                 110
Thr Ser Trp Lys Gln Thr Leu Leu Val Arg Leu Val Pro Val Ser Ala
                115                 120                 125
Leu Leu Ala Ile Thr Tyr His Ala Trp His Leu Thr Leu Pro Asn Lys
            130                 135                 140
Ser Ser Phe Tyr Gly Ser Cys Ile Leu Trp Tyr Ala Cys Pro Val Leu
        145                 150                 155
Ala Ile Leu Trp Leu Gly Ala Gly Glu Tyr Ile Leu Arg Arg Pro Val
    160                 165                 170
Ala Val Leu Leu Ser Ile Val Ile Pro Ser Val Tyr Leu Cys Trp Ala
175                 180                 185                 190
Asp Ile Val Ala Ile Ser Ala Gly Thr Trp His Ile Ser Leu Arg Thr
                195                 200                 205
Ser Thr Gly Lys Met Val Val Pro Asp Leu Pro Val Glu Glu Cys Leu
            210                 215                 220
Phe Phe Thr Leu Ile Asn Thr Val Leu Val Phe Ala Thr Cys Ala Ile
        225                 230                 235
Asp Arg Ala Gln Ala Ile Leu His Leu Tyr Lys Ser Ser Val Gln Asn
    240                 245                 250
Gln Asn Pro Lys Gln Ala Ile Ser Leu Phe Gln His Val Lys Glu Leu
255                 260                 265                 270
Ala Trp Ala Phe Cys Leu Pro Asp Gln Met Leu Asn Asn Glu Leu Phe
                275                 280                 285
Asp Asp Leu Thr Ile Ser Trp Asp Ile Leu Arg Lys Ala Ser Lys Ser
            290                 295                 300
Phe Tyr Thr Ala Ser Ala Val Phe Pro Ser Tyr Val Arg Gln Asp Leu
        305                 310                 315
Gly Val Leu Tyr Ala Phe Cys Arg Ala Thr Asp Asp Leu Cys Asp Asp
    320                 325                 330
Glu Ser Lys Ser Val Gln Glu Arg Arg Asp Gln Leu Asp Leu Thr Arg
335                 340                 345                 350
Gln Phe Val Arg Asp Leu Phe Ser Gln Lys Thr Ser Ala Pro Ile Val
                355                 360                 365
Ile Asp Trp Glu Leu Tyr Gln Asn Gln Leu Pro Ala Ser Cys Ile Ser
            370                 375                 380
Ala Phe Arg Ala Phe Thr Arg Leu Arg His Val Leu Glu Val Asp Pro
        385                 390                 395
Val Glu Glu Leu Leu Asp Gly Tyr Lys Trp Asp Leu Glu Arg Arg Pro
    400                 405                 410
Ile Leu Asp Glu Gln Asp Leu Glu Ala Tyr Ser Ala Cys Val Ala Ser
415                 420                 425                 430
Ser Val Gly Glu Met Cys Thr Arg Val Ile Leu Ala Gln Asp Gln Lys
                435                 440                 445
Glu Asn Asp Ala Trp Ile Ile Asp Arg Ala Arg Glu Met Gly Leu Val
            450                 455                 460
Leu Gln Tyr Val Asn Ile Ala Arg Asp Ile Val Thr Asp Ser Glu Thr
        465                 470                 475
Leu Gly Arg Cys Tyr Leu Pro Gln Gln Trp Leu Arg Lys Glu Glu Thr
    480                 485                 490
Glu Gln Ile Gln Gln Gly Asn Ala Arg Ser Leu Gly Asp Gln Arg Leu
495                 500                 505                 510
Leu Gly Leu Ser Leu Lys Leu Val Gly Lys Ala Asp Ala Ile Met Val
                515                 520                 525
Arg Ala Lys Lys Gly Ile Asp Lys Leu Pro Ala Asn Cys Gln Gly Gly
            530                 535                 540
Val Arg Ala Ala Cys Gln Val Tyr Ala Ala Ile Gly Ser Val Leu Lys

545                 550                 555
Gln Gln Lys Thr Thr Tyr Pro Thr Arg Ala His Leu Lys Gly Ser Glu
    560                 565                 570
Arg Ala Lys Ile Ala Leu Leu Ser Val Tyr Asn Leu Tyr Gln Ser Glu
575                 580                 585                 590
Asp Lys Pro Val Ala Leu Arg Gln Ala Arg Lys Ile Lys Ser Phe Phe
                595                 600                 605
Val Asp

<210> SEQ ID NO:4
<211> 582
<212> 3
<213> 3
<223> Pm 66426DA
<223> Type of sequence: deduced amino acid sequence encoded
by the carB gene of B. trispora.

<400>

Met Ser Asp Gln Lys Lys His Ile Val Val Ile Gly Ala Gly Ile Gly
1               5                   10                  15
Gly Thr Ala Thr Ala Ala Arg Leu Ala Arg Glu Gly Phe Arg Val Thr
            20                  25                  30

Val Val Glu Lys Asn Asp Phe Ser Gly Gly Arg Cys Ser Phe Ile His
        35                  40                  45

His Asp Gly His Arg Phe Asp Gln Gly Pro Ser Leu Tyr Leu Met Pro
    50                  55                  60

Lys Leu Phe Glu Asp Ala Phe Ala Asp Leu Asp Glu Arg Ile Gly Asp
65                  70                  75                  80

His Leu Asp Leu Leu Arg Cys Asp Asn Asn Tyr Lys Val His Phe Asp
                85                  90                  95

Asp Gly Asp Ala Val Gln Leu Ser Ser Asp Leu Thr Lys Met Lys Gly
            100                 105                 110

Glu Leu Asp Arg Ile Glu Gly Pro Leu Gly Phe Gly Arg Phe Leu Asp
        115                 120                 125

Phe Met Lys Glu Thr His Val His Tyr Glu Gln Gly Thr Phe Ile Ala
    130                 135                 140

Ile Lys Arg Asn Phe Glu Thr Ile Trp Asp Leu Ile Arg Leu Gln Tyr
145                 150                 155                 160

Val Pro Glu Ile Phe Arg Leu His Leu Phe Gly Lys Ile Tyr Asp Arg
                165                 170                 175

Ala Ser Lys Tyr Phe Gln Thr Lys Lys Met Arg Met Ala Phe Thr Phe
            180                 185                 190

Gln Thr Met Tyr Met Gly Met Ser Pro Tyr Asp Ala Pro Ala Val Tyr
        195                 200                 205
Ser Leu Leu Gln Tyr Thr Glu Phe Ala Glu Gly Ile Trp Tyr Pro Arg
    210                 215                 220
Gly Gly Phe Asn Met Val Val Gln Lys Leu Glu Ser Ile Ala Ser Lys
225                 230                 235                 240
Lys Tyr Gly Ala Glu Phe Arg Tyr Gln Ser Pro Val Ala Lys Ile Asn
                245                 250                 255
Thr Val Asp Lys Asp Lys Arg Val Thr Gly Val Thr Leu Glu Ser Gly
            260                 265                 270
Glu Val Ile Glu Ala Asp Ala Val Val Cys Asn Ala Asp Leu Val Tyr
        275                 280                 285
Ala Tyr His His Leu Leu Pro Pro Cys Asn Trp Thr Lys Lys Thr Leu
    290                 295                 300
Ala Ser Lys Lys Leu Thr Ser Ser Ser Ile Ser Phe Tyr Trp Ser Met
305                 310                 315                 320
Ser Thr Lys Val Pro Gln Leu Asp Val His Asn Ile Phe Leu Ala Glu
                325                 330                 335
Ala Tyr Lys Glu Ser Phe Asp Glu Ile Phe Asn Asp Phe Gly Leu Pro
            340                 345                 350
Ser Glu Ala Ser Phe Tyr Val Asn Val Pro Ser Arg Ile Asp Glu Ser
        355                 360                 365
Ala Ala Pro Pro Asn Lys Asp Ser Ile Ile Val Leu Val Pro Ile Gly
    370                 375                 380
His Met Lys Ser Lys Thr Gly Asn Ser Ala Glu Glu Asn Tyr Pro Glu
385                 390                 395                 400
Leu Val Asn Arg Ala Arg Lys Met Val Leu Glu Val Ile Glu Arg Arg
                405                 410                 415
Leu Gly Val Asn Asn Phe Ala Asn Leu Ile Glu His Glu Glu Val Asn
            420                 425                 430
Asp Pro Ser Val Trp Gln Ser Lys Phe Asn Leu Trp Arg Gly Ser Ile
        435                 440                 445
Leu Gly Leu Ser His Asp Val Phe Gln Val Leu Trp Phe Arg Pro Ser
    450                 455                 460
Thr Lys Asp Ser Thr Asn Arg Tyr Asp Asn Leu Phe Phe Val Gly Ala
465                 470                 475                 480
Ser Thr His Pro Gly Thr Gly Val Pro Ile Val Leu Ala Gly Ser Lys
                485                 490                 495
Leu Thr Ser Asp Gln Val Cys Lys Ser Phe Gly Gln Asn Pro Leu Pro
            500                 505                 510
Arg Lys Leu Gln Asp Ser Gln Lys Lys Tyr Ala Pro Glu Gln Thr Arg
        515                 520                 525
Lys Thr Glu Ser His Trp Ile Tyr Tyr Cys Leu Ala Cys Tyr Phe Val
    530                 535                 540

Thr Phe Leu Phe Phe Tyr Phe Phe Pro Arg Asp Asp Thr Thr Thr Pro
545                 550                 555                 560
Ala Ser Phe Ile Asn Gln Leu Leu Pro Asn Val Phe Gln Gly Gln Asn
                565                 570                 575
Ser Asn Asp Ile Arg Ile
            580

<210> SEQ ID NO: 5
<211> 20
<212> DNA
<213> artificial sequence
<223> PCR primer

<400>

CGCGCCGACT GCCATTGACT                    20

<210> SEQ ID NO. 6
<211> 19
<212> DNA
<213> artificial sequence
<223> PCR primer

<400>

CACGCACGCC GCCTTGACA                     19

<210> SEQ ID NO: 7
<211> 28
<212> DNA
<213> artificial sequence
<223> oligonucleotide for inserting the cutting site NcoI in
the carB gene

<400>

CTATTTTAAT CCCATGGCTG ATCAAAAG           28

<210> SEQ ID NO: 8
<211> 28
<212> DNA
<213> artificial sequence

<223> oligonucleotide for inserting the cutting site NcoI in
the carB gene

<400>

CTTTTGATCA GCCATGGGAT TAAAATAG          28

<210> SEQ ID NO: 9
<211> 31
<212> DNA
<213> artificial sequence
<223> oligonucleotide for inserting the cutting site NcoI in
the carRP gene

<400>

CTTTTTATTT TATCTCCATG GCAATACTCA C      31

<210> SEQ ID NO:10
<211> 31
<212> DNA
<213> artificial sequence
<223> oligonucleotide for inserting the cutting site NcoI in
the carRP gene

<400>

GTGAGTATTG CCATGGAGAT AAAATAAAAA G      31

## Claims

1. A DNA compound isolated from *B. trispora* that contains the *carRP* and *carB* genes, which is defined by the restriction map shown in Fig. 1 and codes for enzymes involved in the biosynthetic pathway of β-carotene.

2. A DNA sequence identifièd as SEQ ID NO:1, which includes the *carRP* gene of *B*. *trispora*, that codes for the enzyme lycopene cyclase/phytoene synthase.

3. A DNA sequence identified as SEQ ID NO:2, which includes the *carB* gene of *B*. *trispora*, that codes for the enzyme phytoene dehydrogenase.

4. Nucleotide sequences **characterized in that** they are capable of hybridization, under restrictive conditions, with the DNA compounds of claims 1 to 3 and express one of the enzymes that are encoded by the *carRP* and *carB* genes, except the sequences of *P. blakesleeanus, M. circinelloides* and *N. crassa*.

5. An amino acid sequence identified as SEQ ID NO:3, which corresponds to the bifunctional enzyme lycopene cyclase / phytoene synthase encoded by the *carRP* gene of *B. trispora*.

6. An amino acid sequence identified as SEQ ID NO:4, which corresponds to the enzyme phytoene dehydrogenase encoded by the *carB* gene of *B. trispora*.

7. Vectors bearing the DNA compounds described in claims 1 to 4 or fragments thereof that code for one of the enzymes encoded by the respective complete sequences.

8. Vectors as claimed in claim 7 that express the *carRP* gene under the control of expression signals different from those of the gene itself.

9. Vectors as claimed in claim 7 that express the *carB* gene under the control of expression signals different from those of the gene itself.

10. Vectors as claimed in claims 8 and 9, **characterized in that** the expression signals are obtained from *B*. *trispora*.

11. Vectors as claimed in any one of claims 7 to 10, **characterized in that** they consist of a plasmid.

12. The plasmid as claimed in claim 11, **characterized in that** it consists of pALBT9, which includes the *carRP* gene.

13. The plasmid as claimed in claim 11, **characterized in that** it consists of pALBT52, which includes the *carB* gene.

14. The plasmid as claimed in claim 11, **characterized in that** it consists of pALBT83, which includes the *carRP* and *carB* genes.

15. The plasmid as claimed in claim 11, **characterized in that** it consists of pALBT84, which includes the *carRP* gene.

16. The plasmid as claimed in claim 11, **characterized in that** it consists of pALBT85, which includes the *carB* gene.

17. The plasmid as claimed in claim 11, **characterized in that** it consists of pALBT57, which includes the *ble*^{*R*} gene expressed under the control of the promoter of the *carRP* gene.

18. The plasmid as claimed in claim 11, **characterized in that** it consists of pALBT58, which includes the *ble*^{*R*} gene expressed under the control of the promoter of the *carB* gene.
